Europäisches Patentamt

European Patent Office

Office européen des brevets

(11) Publication number: **0 319 278 B1**

## EUROPEAN PATENT SPECIFICATION

(45) Date of publication of patent specification: **26.08.92** (51) Int. Cl.⁵: **A61M 5/14**

(21) Application number: **88311354.0**

(22) Date of filing: **30.11.88**

(54) Disposable cassette for a medication infusion system.

(30) Priority: **01.12.87 US 127333**

(43) Date of publication of application:
**07.06.89 Bulletin 89/23**

(45) Publication of the grant of the patent:
**26.08.92 Bulletin 92/35**

(84) Designated Contracting States:
**DE FR GB IT NL SE**

(56) References cited:
**EP-A- 0 223 580**
**FR-A- 2 367 503**
**GB-A- 2 107 800**
**GB-A- 2 126 666**

(73) Proprietor: **PACESETTER INFUSION LTD. doing business as MINIMED TECHNOLOGIES**
**12884 Bradley Avenue**
**Sylmar California 91342(US)**

(72) Inventor: **Pelmulder, John P.**
**10825 Farralone Avenue**
**Chatsworth California 91311(US)**
Inventor: **Gorton, Lanny A.**
**10728 Wescott Avenue**
**Sunland California 91040(US)**

(74) Representative: **Rees, David Christopher et al**
**Kilburn & Strode 30 John Street**
**London WC1N 2DD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 319 278 B1

## Description

The present invention relates generally to an electromechanical system for continuously infusing medication into a patient, and more particularly to a disposable cassette for installation onto and use with a main pump unit containing a prime mover for operating the fluid pump.

In the past there have been two primary techniques which have been used to deliver drugs which may not be orally ingested to a patient. The first such technique is through an injection, or shot, using a syringe and needle which delivers a large dosage at relatively infrequent intervals to the patient. This technique is not always satisfactory, particularly when the drug being administered is potentially lethal, has negative side effects when delivered in a large dosage, or must be delivered more or less continuously to achieve the desired therapeutic effect. This problem results in smaller injections being given at more frequent intervals, a compromise approach not yielding satisfactory results.

The second technique involves administering a continuous flow of medication to the patient, typically through an IV bottle. Medication may also be delivered through an IV system with an injection being made into a complex maze of IV tubes, hoses, and other paraphernalia. With drop counters being used to meter the amount of bulk fluid delivered, many medications still end up being administered in a large dosage through an injection into the IV lines, although the medications may be diluted somewhat by the bulk fluid.

As an alternative to these two techniques of administering medication to a patient, the relatively recent addition of medication infusion pumps has come as a welcome improvement. Medication infusion pumps are used to administer drugs to a patient in small, metered doses at frequent intervals or, alternatively, in the case of some devices, at a low but essentially continuous rate. Infusion pump therapy may be electronically controlled to deliver precise, metered doses at exactly determined intervals, thereby providing a beneficial gradual infusion of medication to the patient. In this manner, the infusion pump is able to mimic a natural process whereby chemical balances are maintained more precisely by operating on a continuous time basis.

GB-A-2107800 discloses a disposable cassette for use with a medication infusion system, as set out in the first part of claim 1, having an inlet passageway, an outlet passageway, a cylinder and diaphragm means having a valve portion being selectively deformable to prevent the flow of fluids between the inlet passageway and a second aperture and said second aperture and the outlet passageway respsectively.

One of the requirements of a medication infusion system is dictated by the important design consideration of disposability. Since the portion of the device through which the medication is pumped must be sterile, in most applications of modern medication infusion equipment, some portions of the equipment are used only once and then disposed of, typically at regular intervals such as once daily. It is therefore desirable that the fluid pump portion of the infusion pump device should be disposable, with the fluid pump being designed as an attachable cassette which is of inexpensive design, and which is easily installable onto the main pump unit.

It is clearly desirable to have a simple disposable cassette design to minimise the cost of its construction, using the minimum number of parts necessary in its design. The design of the cassette should be mass produceable, and yet result in a uniform cassette which is capable of delivering liquid medication or other therapeutic fluids with a high degree of accuracy. The cassette should include more than just a fluid pump; other features which have formerly been included in peripheral devices should also be included in the cassette.

It is therefore the primary object of the present invention to provide a disposable cassette for use with a main pump unit, of a design retaining all of the advantages of such devices known in the past, while also affording a high degree of precision and accuracy, with the degree of precision remaining constant throughout the life of the cassette.

It is a further object to provide a cassette having means for conveniently and easily priming the pump. It is a further object to provide a design including a bubble trap to prevent the frequent shutdowns and alarms exhibited by presently known pumps.

According to the present invention, there is provided a disposable cassette for use with a medication infusion system which comprises a cassette body (100) having an upper surface (102) with first and second apertures (110,118) extending therethrough, the upper surface (102) having an inlet passageway (128) and an outlet passageway (134); a cylinder (112) below the upper surface of the cassette, one end of the cylinder (112) being sealingly attached to the bottom of the upper surface (102), the other end of the cylinder (112) being open, the second aperture (118) being in communication with the interior of the cylinder (112); and diaphragm means (170) for sealing the open top sides of the inlet and outlet passageways (128,134), the diaphragm means (170) having a valve portion (178) which forms a cavity (180) over a portion of the upper surface of the cassette body, whereby the cavity (180) is in fluid communication with the inlet passageway (128), the valve portion (178) being selectively deformable to prevent the flow of fluids between the inlet passageway (128) and the second aperture (118); characterised in that: the inlet and outlet passageways (128,134) are recessed in the upper surface (102); the inlet passageway (128) extends between the first aperture (110) and a location near to the second aperture (118); the outlet passageway (134) extends between the location near to the second aperture (118) and a third aperture (142) in the upper surface (102); the inlet and outlet passageways (128,134) are open on the top side of the upper surface (102); a piston (260) is located in the cylinder (112) and is operable for varying the fluid volume contained in the cylinder (112) by alternatively drawing fluid into the cylinder (112) and forcing fluid out of the cylinder (112) through the second aperture (118); and cavity (180) extends over the said other end of the inlet passageway (128), the second aperture (118) and the said one end of the outlet passageway (134), whereby the cavity (180) is in the fluid communication with the second aperture (118) leading to the cylinder (112), and the outlet passageway (134); and the valve portion (178) is also selectively deformable to prevent the flow of fluids between the second aperture (128) and outlet passageway (134) during normal working of the system.

Preferably the valve portion of the diaphragm means comprises a flexible, resilient dome which forms the cavity. Preferably, the dome is integral with the diaphragm means, the diaphragm means being located around the dome. Preferably, the cassette body is of a one-piece moulded construction.

There may also be a bubble trap disposed below the upper surface of the cassette body, the bubble trap preferably comprising a bubble chamber located below the upper surface of the cassette body the bubble chamber being open at the bottom; a siphon tube located in the bubble chamber extending from the bottom of the upper surface to a position somewhat above the bottom of the bubble chamber the siphon tube having a bore which is open at the bottom of the siphon tube and communicates at the top with the first aperture; and a bubble chamber cap for sealing the open bottom of the bubble chamber, the bubble chamber, the bubble chamber cap including an inlet tube extending up into the bubble chamber from the bottom to a level above the bottom of the siphon tube.

Preferably, there is a slightly raised border which surrounds the inlet passageway the second aperture and the outlet passageway, and includes a rectangular portion spaced from and surrounding the said other end of the inlet passageway, the second aperture, and the said one end of the outlet passageway the rectangular portion being broken only at the locations where the inlet passageway and the outlet passageway extend out of the rectangular portion, the border co-operating with the diaphragm means for sealing purposes. Preferably, there is also a first raised segment located between the said other end of the inlet passageway and the second aperture and a second raised segment located between the said one end of the outlet passageway and the second aperture; the two raised segments being located below the valve portion and being wider than the cavity below the valve portion; the first raised segment cooperating with the valve portion to prevent selectively the flow of fluids between the inlet passageway and the second aperture, and the second raised segment cooperating with the valve portion to prevent selectively the flow of fluids between the second aperture and the outlet passageway.

The system may also include means for selectively preventing the flow of fluid through the cassette prior to its installation, for example to avoid inadvertent flow, the preventing means being operable to allow the flow of fluid through the cassette prior to installation in order to prime the cassette, the preventing means preferably comprising an outlet tube for receiving fluid pumped form the cassette body; the outlet tube being fixedly attached to the cassette body by outlet tube mounting means located below the upper surface of the cassette body, the outlet tube having one end communicating with the third aperture in order to receive the fluid; and means for selectively clamping the outlet tube to prevent fluid from flowing. The clamping means may comprise a slide latch having a tear-shaped aperture the tear-shaped aperture having a wider end and a narrower end; and slide latch support means located below the upper surface of the cassette body, supporting the slide latch for slideable movement with the tear-shaped aperture located under the outlet tube mounting means, the outlet tube extending through the tear-shaped aperture, the slide latch having a first position in which the outlet tube extends through the wider end of the tear-shaped aperture, allowing fluid to flow through the outlet tube, the slide latch also having a second position in which the outlet tube extends through the narrower end of the tear-shaped aperture thereby constricting the outlet tube to prevent fluid from flowing though the outlet tube.

There may also be a retainer cap for installation over the upper surface of the diaphragm means and cassette body, the retainer cap retaining the diaphragm means in a sealing relationship over the upper surface of the cassette body, the retainer cap having an aperture allowing the valve portion of the diaphragm means to project therethrough, permitting access to the valve portion so that it may be operated.

A cassette in accordance with the invention enjoys all the advantages of known designs but is also capable of accurate mass-production and may include many features which are peripheral to known systems.

Several other additional features may be desirable in the design of a cassette and a main pump unit making up the infusion system. Examples of such features are a wide range in the volume which may be pumped over a particular period of time, the ability to gravity flow fluid through the device, and safeguards preventing accidental flow through the cassette when it is removed from the main pump unit. No pump known in the art even comes close to combining these features, or even a majority of the features enumerated above whereas they may be present in the system according to the invention.

A cassette of the preferred embodiment may also include additional devices such as pressure sensing means and bubble detecting means, which are separate assemblies in known medication infusion systems. Despite the inclusion of more features than contained in any known design, the cassette of the present invention uses a minimum number of parts, all of the parts being inexpensive in construction yet affording the assembled cassette the high degree of accuracy which must be retained.

The cassette of the present invention may have other advantages. Specifically, the cassette of the present invention is less costly than many known systems, and is easier to use. It accomplishes all these objects while retaining the advantages of reliability, durability, and safety of operation, and without incurring any relative disadvantage. All the advantages of the present invention will result in a superior medication infusion system having a number of advantages making the system a highly desirable alternative to systems presently available.

The disadvantages and limitations of the background art discussed above are overcome by the present invention. With this invention, a disposable cassette having only seven components is described. The cassette uses a highly accurate and reliable piston-type fluid pump, which presents a number of operational advantages over competing designs which use a diaphragm pump, since diaphragm pumps generally provide somewhat less accuracy than piston pumps. The pump also uses an active valve design of unparalleled accuracy, simplicity, and accuracy of operation.

A bubble trap may be included in the cassette for removing air bubbles which may be introduced into the system by the fluid supply apparatus. The cassette may have standard luer fittings on inlet and outlet tubes. In the preferred embodiment, the cassette also includes both a pressure diaphragm for enabling pressure sensing of the outlet line, and a bubble detector for ensuring that fluid supplied to a patient is virtually bubble-free.

The cassette of the present invention can provide a wide range in the volume which may be pumped over a particular period of time. A highly desirable addition is the ability to gravity-flow fluid through the device, both when priming the cassette and in the event of a power failure to continue fluid infusion. Built in safeguards of the system may effectively prevent accidental flow through the cassette when it is removed from the main pump unit.

The cassette of the present invention includes a pump which can provide a high degree of accuracy and wide range of infusion rates, may be conveniently and easily primed, and may include a bubble trap to prevent the frequent shutdowns and alarms exhibited by presently known pumps. The ability to gravity-flow fluids through the cassette is possible, while accidental flow through the cassette when the cassette is removed from the main pump unit is effectively prevented. The cassette of the preferred embodiment also includes both pressure sensing means and bubble detecting means in the cassette itself rather than in peripheral devices.

Despite the inclusion of more features than contained in any competing design, the cassette of the present invention uses only seven parts, all of which parts are of inexpensive yet highly precise constructions. The cassette of the present invention is of a design and construction which enables it to be produced more economically than known systems, and also provides an ease of use rivaling the best of such known systems. It provides all of the advantages and features mentioned above, which are not all contained in any competing cassette. The cassette accomplishes all of these objectives and advantages in a manner which will retain all of the advantages of reliability, durability, and safety of operation, without incurring any relative disadvantage whatsoever. Accordingly, it will be perceived that the cassette enables a superior medication infusion system having a number of advantages making the system a highly desirable alternative to systems presently available.

The invention may be carried into practice in various ways and some embodiments will now be described by way of example with reference to the accompanying drawings. In this description of the preferred embodiments, a uniform directional system is used in which front, back, top, bottom, left and right are indicated with respect to the operating position of the cassette and main pump unit when viewed from the front of the main pump unit. In the accompanying drawings:

Figure 1 is a top plan view of a disposable cassette body showing most of the fluid path through the cassette;

Figure 2 is a top plan view of a disposable cassette body shown in Figure 1;

Figure 3 is a rear-elevation (inverted) of the cassette body shown in Figures 1 and 2;

Figure 4 is a bottom view of the cassette body shown in Figures 1 to 3;

Figure 5 is a right side view (inverted) of the cassette body shown in Figures 1 to 4;

Figure 6 is a left side view of the cassette body showing in Figures 1 to 5;

Figure 7 is a partially cutaway view from the front of the cassette body shown in Figures 1 to 6, showing the bubble trap used to remove air bubbles from the fluid supplied to the cassette;

Figure 8 is a partially cutaway view from the right side (inverted) of the cassette body shown in Figures 1 to 6, showing the cylinder of the fluid pump contained in the cassette;

Figure 9 is a top plan view of a valve diaphragm used to seal the passageways on the top surface of the cassette body showing in Figure 1;

Figure 10 is a bottom view of the valve diaphragm shown in Figure 9;

Figure 11 is a vertical section through the valve diaphragm shown in Figures 9 and 10, viewed from the rear;

Figure 12 is a partially cutaway view from the right side of the valve diaphragm shown in Figures 9 and 10;

Figure 13 is a top plan view of a valve diaphragm retainer used to retain the valve diaphragm shown in Figures 9 to 12;

Figure 14 is a bottom view of the valve diaphragm retainer shown in Figure 13;

Figure 15 is a rear elevation of the valve diaphragm retainer shown in Figures 13 and 14;

Figure 16 is a front elevation of the valve diaphragm retainer shown in Figures 13 to 15;

Figure 17 is a right side view of the valve diaphragm retainer shown in Figures 13 to 16;

Figure 18 is a left side view of the valve diaphragm retainer shown in Figures 13 to 17;

Figure 19 is a vertical section through the valve diaphragm retainer shown in Figures 13 to 18, viewed from the front;

Figure 20 is a partially cutaway view from the left side of the valve diaphragm retainer shown in Figures 13 to 19;

Figure 21 is a partially cutaway view from the right side of the valve diaphragm retainer shown in Figures 13 to 20;

Figure 22 is a top view of a bubble chamber cap;

Figure 23 is a bottom view of the bubble chamber cap shown in Figure 22;

Figure 24 is a left side view of the bubble chamber cap shown in Figures 22 and 23;

Figure 25 is a cutaway view from the rear of the bubble chamber cap shown in Figures 22 to 24;

Figure 26 is a cutaway view (inverted) from the right side of the bubble chamber cap shown in Figures 22 to 24;

Figure 27 is a top plan view of a slide latch used both the lock the cassette in place on a main pump unit, and to pinch off the IV outlet line prior to installation on the main pump unit;

Figure 28 is a right side view of the slide latch shown in Figure 27;

Figure 29 is a bottom view of the slide latch shown in figures 27 and 28;

Figure 30 is a rear view of the slide latch shown in Figures 27 to 29;

Figure 31 is a front elevation of the slide latch shown in Figures 27 to 30;

Figure 32 is a cutaway view (inverted) from the left side of the slide latch shown in Figures 27 to 31;

Figure 33 is a side view of the piston cap and boot seal, which function both as a piston and as a bacterial seal;

Figure 34 is a view from above of the piston cap and boot seal shown in Figure 33;

Figure 35 is a view from below of the piston cap and boot seal shown in Figures 33 and 34;

Figure 36 is a vertical section through the piston cap and boot seal shown in Figures 33 to 35;

Figure 37 is a rear view of a piston for insertion into the piston cap and boot seal shown in Figures 33 to 36;

Figure 38 is a front view of the piston shown in Figure 37;

Figure 39 is a top view of the piston shown in Figures 37 and 38;

Figure 40 is a left side view of the piston shown in Figures 37 to 39;

Figure 41 is a bottom view of the piston shown in Figures 37 to 40;

Figure 42 is a cutaway view (inverted) from the right side of the piston shown in Figures 37 to 41;

Figure 43 is a top plan view of an assembled cassette using the components shown in Figures 1 to 42, with the slide latch in the closed position;

Figure 44 is a bottom view of the assembled cassette shown in Figure 43;

Figure 45 is a front view of the assembled cassette shown in Figures 43 and 44;

Figure 46 is a rear view (inverted) of the assembled cassette shown in Figures 43 to 45;

Figure 47 is a left side view of the assembled cassette shown in Figures 43 to 46;

Figure 48 is a right side view of the assembled cassette shown in Figures 43 to 47;

Figure 49 is a left side view of the latch head used to capture and actuate the piston;

Figure 50 is a right side view of the latch head shown in Figure 49;

Figure 51 is a bottom view of the latch head shown in Figures 49 and 50;

Figure 52 is top view of the latch head shown in Figures 49 to 51;

Figure 53 is a cutaway view from the right side of the latch head shown in Figures 49 to 52;

Figure 54 is a right side view of the spring retainer to be mounted in the latch head shown in Figures 49 to 52;

Figure 55 is a front view of the spring retainer shown in Figure 54;

Figure 56 is a left side view of the latch jaw to be mounted on the latch head shown in Figures 49 to 52;

Figure 57 is a bottom view of the latch jaw shown in Figure 56;

Figure 58 is a rear view of the latch jaw shown in Figures 56 and 57;

Figure 59 is a left side view of the jaws assembly in the open position, the jaws assembly being made up of the latch head shown in Figures 49 to 52, the spring retainer shown in Figures 54 and 55, the latch jaw shown in Figures 56 to 58, a latch spring, and pins to assemble the various components together;

Figure 60 is a bottom view of the jaws assembly shown in Figure 59, with the jaws assembly being shown in the open position;

Figure 61 is a left side view of the jaws assembly shown in Figures 59 and 60, with the jaws assembly being shown in the closed position (and in the open position in phantom lines);

Figure 62 is a bottom plan view of the main pump unit chassis;

Figure 63 is a front view of the main pump unit chassis shown in Figure 62;

Figure 64 is a top plan view of the main pump unit chassis shown in Figures 62 and 63;

Figure 65 is a rear view (inverted) of the main pump unit chassis shown in Figures 62 to 64;

Figure 66 is a bottom plan view of the cassette guide used to position the cassette of Figures 43 to 48 on the main pump unit;

Figure 67 is a top plan view of the cassette guide shown in Figure 66;

Figure 68 is a front view of the cassette guide shown in Figures 66 and 67;

Figure 69 is a right side view of the cassette guide shown in Figures 66 to 68;

Figure 70 is a side view of the pump shaft on which the jaws assembly shown in Figures 59 to 61 is mounted;

Figure 71 is a right side view of the slide lock used to retain the cassette shown in Figures 43 to 48 in position on the main pump unit;

Figure 72 is a bottom view of the slide lock shown in Figure 71;

Figure 73 is a left side view (inverted) of the slide lock shown in Figures 71 and 72, showing the bevel used to reflect the light beam from the optical light source away from the optical light sensor when the slide lock is in the open position;

Figure 74 is a top view of the slide lock shown in Figures 71 to 73, showing the reflective surface used to reflect the light beam from the optical light source to the optical light sensor when the slide lock is in the closed position;

Figure 75 is a front view of the slide lock shown in Figures 71 to 74;

Figure 76 is a rear view of the slide lock shown in Figures 71 to 75, showing the slanted surfaced used to reflect the light beam away from the corresponding sensor when the slide lock is in the open position;

Figure 77 is a side view of the power module cam used both to drive the pump through the pump shaft shown in Figure 70 and to drive the valve actuators;

Figure 78 is a side view of the power module cam rotated ninety degrees from the view of Figure 77;

Figure 79 is a bottom view of the power module cam shown in Figures 77 and 78;

Figure 80 is a graph showing the inlet and outlet valve positions and the pump displacement plotted against the angular position of the power module cam shown in Figures 77 to 79;

Figure 81 is a front view of the drive assembly including the motor/cam mount, the motor, the power module cam shown in Figures 77 to 79, and the position encoder assembly;

Figure 82 is a top view of the motor/cam mount included in the drive assembly shown in Figure 81;

Figure 83 is a top view of one of the actuator guides used to guide and retain in position the valve actuators for one cassette;

Figure 84 is a side view of the actuator guide shown in Figure 83;

Figure 85 is a front view of a valve actuator;

Figure 86 is a side view of the valve actuator shown in Figure 85;

Figure 87 is a bottom view of the valve actuator shown in Figures 85 and 86;

Figure 88 is a top plan view of a pressure transducer;

Figure 89 is a side view of the pressure transducer shown in Figure 88;

Figure 90 is a bottom view of the pressure transducer shown in Figures 88 and 89;

Figure 91 is a front view of an optical sensor module;

Figure 92 is a side view of the optical sensor module shown in Figure 91;

Figure 93 is a top plan view of the optical sensor module shown in Figures 91 and 92;

Figure 94 is a bottom view of the optical sensor module shown in Figures 91 to 93 showing the optical source and sensor pair for detecting the closed position of the slide lock;

Figure 95 is a section on the line 95-95 in Figure 91 showing the optical sources for detecting the cassette identification bits;

Figure 96 is a section on the line 96-96 in Figure 91 showing the optical sensors for detecting the cassette identification bits, and the optical source and sensor pair for detecting air bubbles in the fluid line;

Figure 97 is a bottom plan view of the elastomeric valve actuator seal used to bias the valve actuators into an upward position;

Figure 98 is a section through the valve actuator seal shown in Figure 97;

Figure 99 is a bottom view of the main pump unit chassis having the various components for one pump mounted thereon, with the slide lock in the open position ready to receive a cassette;

Figure 100 is a bottom view of the main pump unit chassis shown in Figure 99, with the slide lock in the closed position as it would be if a cassette were installed and latched onto the main pump unit;

Figure 101 is a top plan view of the cassette shown in Figures 43 to 49 in the installed position relative to the optical sensor module, with all other parts removed for clarity;

Figure 102 is a side view of the cassette and optical sensor module of Figure 101;

Figure 103 is a section through the cassette and the optical sensor module of Figures 101 and 102, showing a cassette identifying indicia having a logic zero value;

Figure 104 is a section through the cassette and the optical sensor module of Figures 101 and 102, showing a cassette identifying indicia having a logic one value;

Figure 105 is a section on the line 105-105 in Figure 99 showing the slide lock in the open position over the cassette-in-place sensor of the optical sensor module;

Figure 106 is a section on the line 106-106 in Figure 105 showing how the slanted surface reflects the light beam away from the cassette-in-place sensor;

Figure 107 is a section on the line 107-107 in Figure 100 showing the slide lock in the closed position over the cassette-in-place sensor of the optical sensor module, with the light beam being reflected back onto the cassette-in-place sensor;

Figure 108 is a section through the cassette and the optical sensor module of Figures 101 and 102, showing the air-in-line detection apparatus of the preferred embodiment;

Figure 109 is a view similar to Figure 108, but showing a first alternative air-in-line detection apparatus;

Figure 110 is a view similar to Figure 108, but showing a second alternative air-in-line detection apparatus;

Figure 111 is a view similar to Figure 108, but showing a third alternative air-in-line detection apparatus;

Figure 112 is a side view of the main pump unit chassis partly in section, and having the various components for one pump mounted thereon and a cassette installed, showing the pump drive train;

Figure 113 is a vertical section through the pump and valves showing the beginning of the fill cycle;

Figure 114 is a vertical section through the pump and valves showing the beginning of the pump cycle;

Figure 115 is a vertical section through the the pressure plateau, the pressure diaphragm, and the pressure transducer; and

Figure 116 is a second vertical section through the pressure plateau, the pressure diaphragm, and the pressure transducer at right angles to Figure 115.

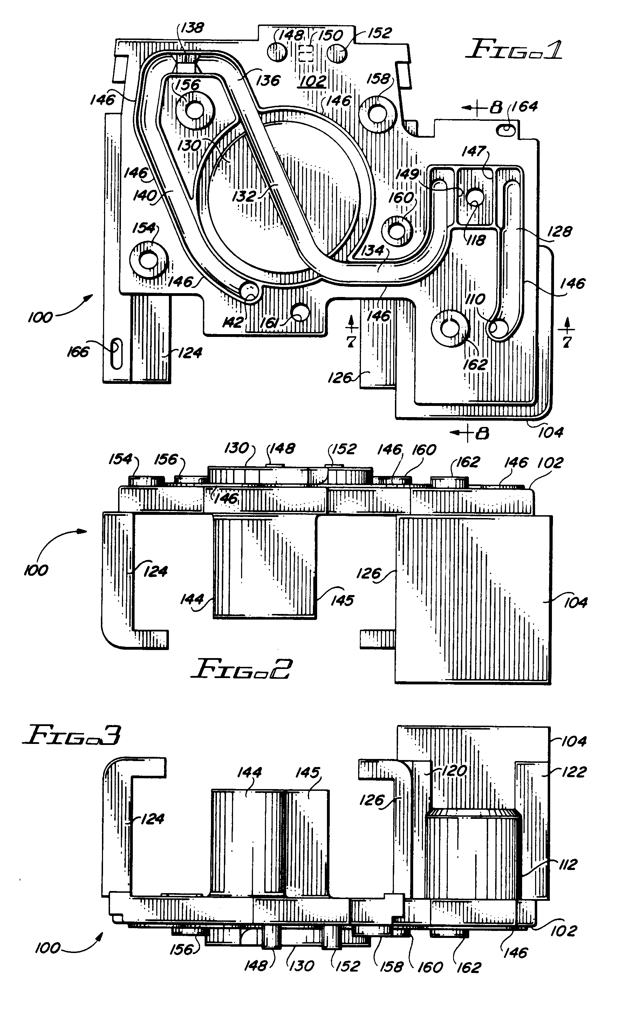

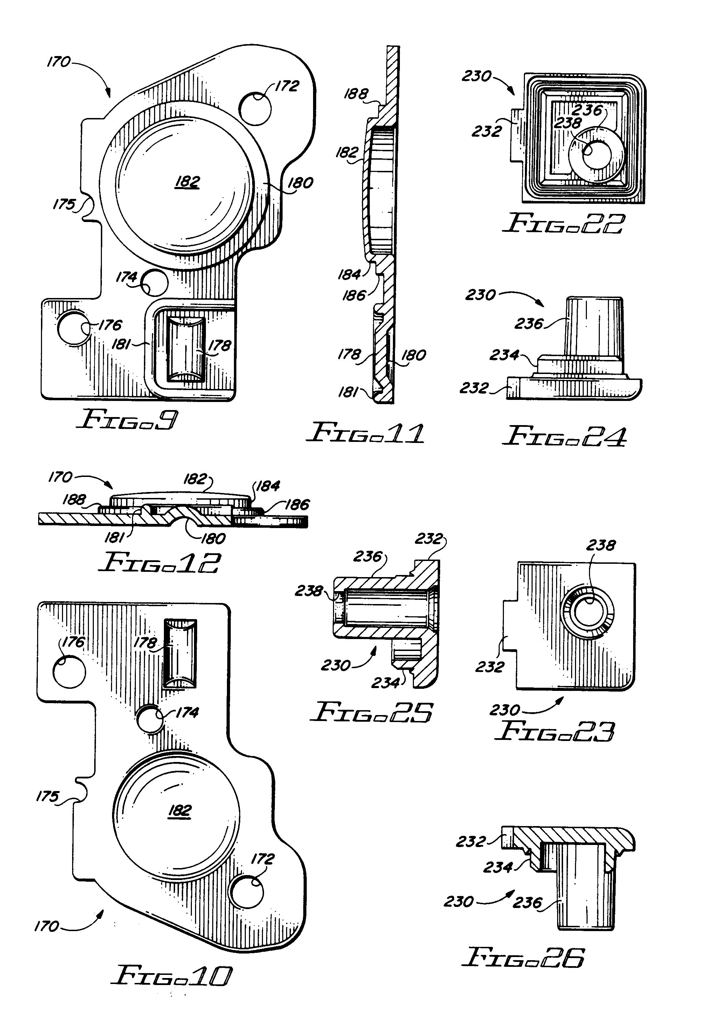

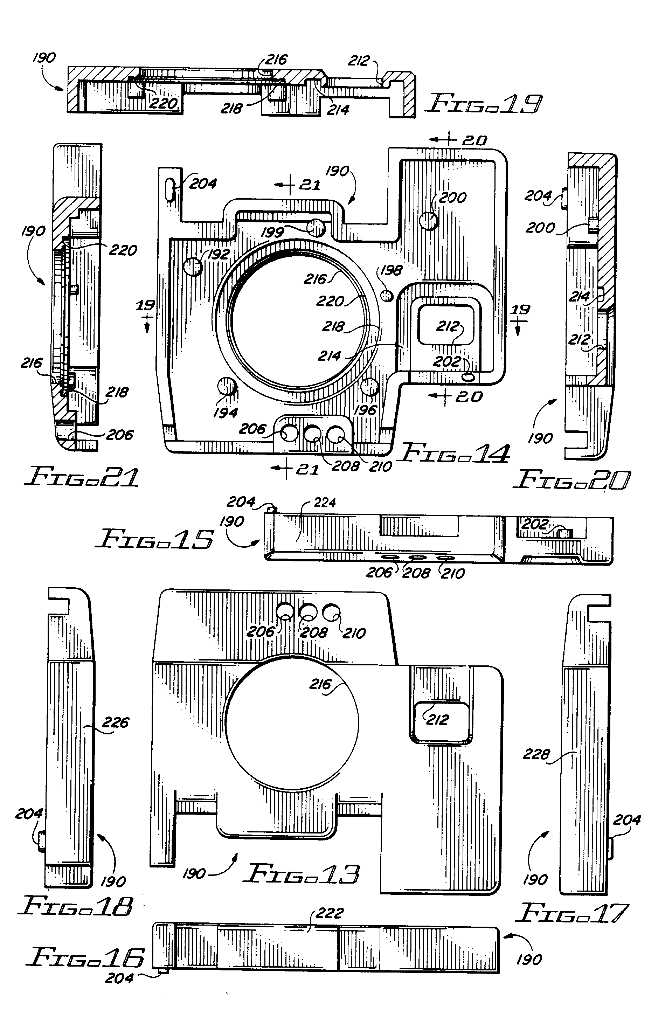

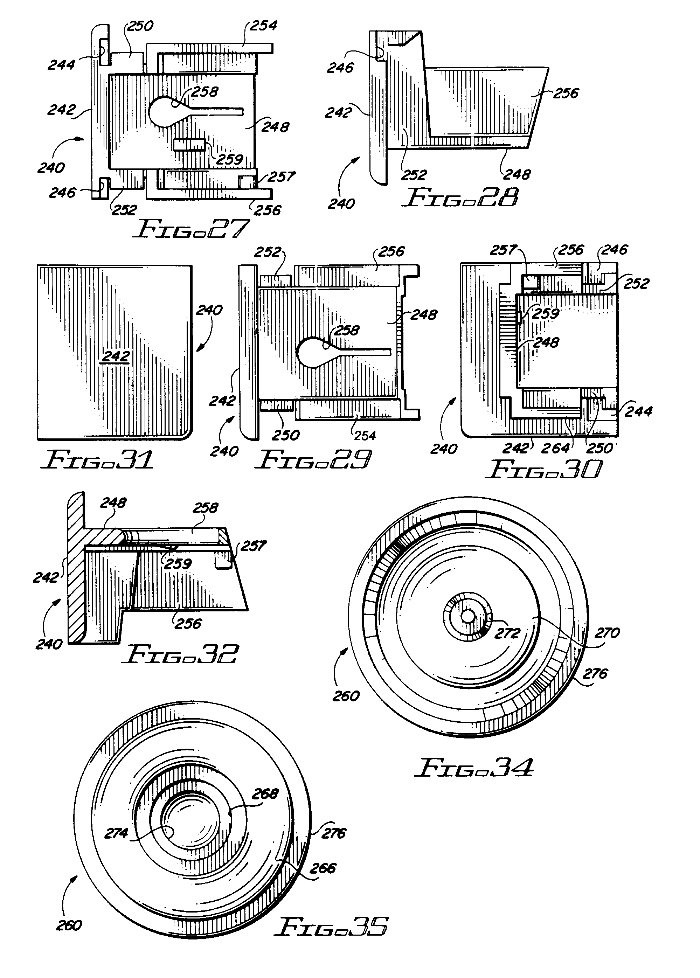

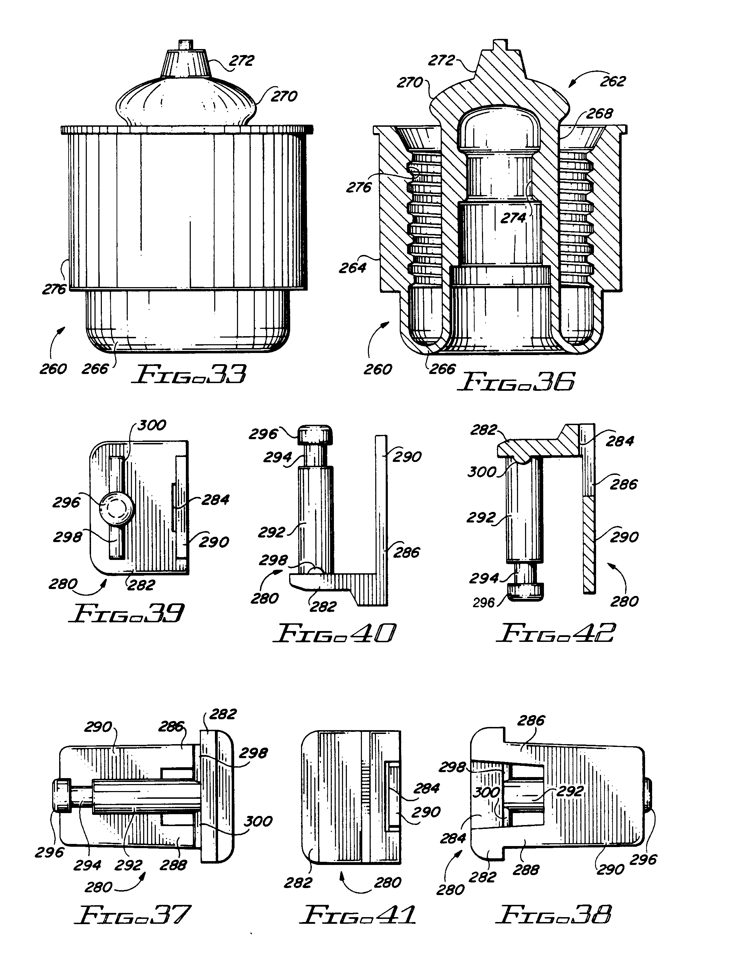

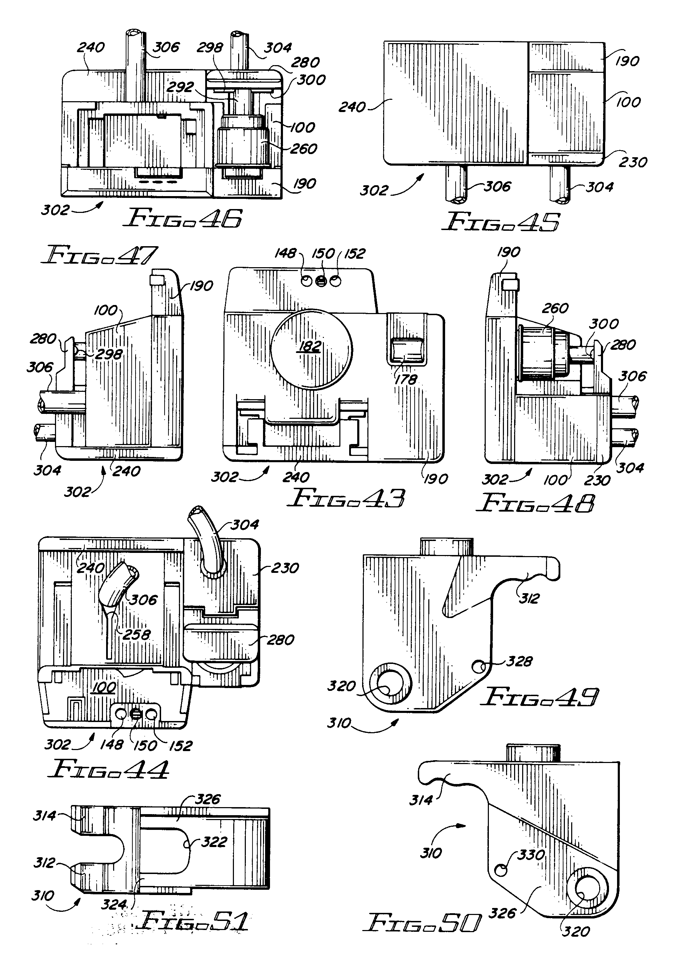

The Cassette The preferred embodiment of the cassette of the present invention includes all of the features described above in a single compact disposable cassette constructed of seven parts. Prior to discussion of the construction and operation of the cassette, it is advantageous to discuss the construction and configuration of the seven components included in the cassette. The first of these components and the one around which the other six components are assembled is a cassette body 100, which is shown in Figures 1 to 8. The cassette body 100 has an upper surface portion 102 which is essentially flat with a number of protrusions and indentations located in the top surface thereof (Figure 1). The upper surface portion 102 has a thickness sufficient to accommodate the indentations mentioned above, some of which are fluid passageways which will be discussed below.

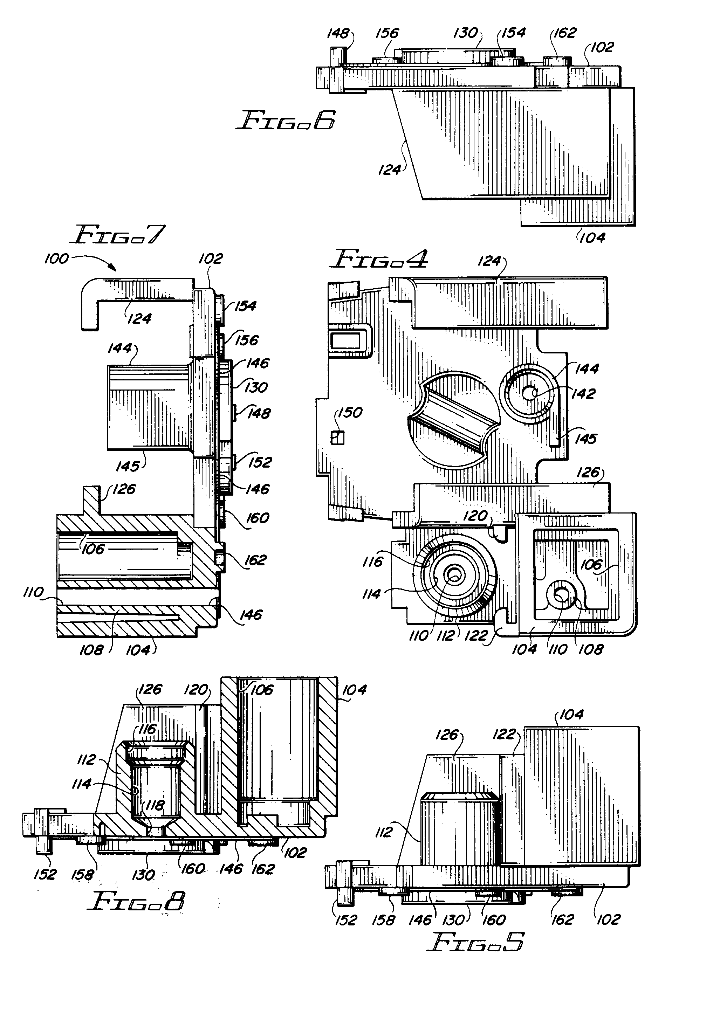

Referring generally to Figures 1 to 8, a bubble trap 104 is located at the front right corner of the cassette body 100 below the upper surface portion 102. The bubble trap 104 is essentially square in cross-section (Figure 4). It includes a bubble chamber 106 which is open at the bottom and closed at the top by the bottom of the upper surface portion 102 of the cassette body 100.

A siphon tube 108 is located in the bubble chamber 106; the siphon tube 108 has a bore 110 leading from the bottom of the bubble chamber 106 to the top of the upper surface portion 102 of the cassette body 100.

Located behind the bubble trap 104 below the upper surface portion 102 of the cassette body 100 on the right side thereof is a pump cylinder 112 (Figures 2 to 5, 8). The pump cylinder 112 does not extend downwards as far as does the bubble trap 104. The pump cylinder 112 is open at its bottom end and is arranged and configured to receive a piston which will be discussed below. The inner configuration of the pump cylinder 112 includes a main diameter bore 114, with a greater diameter bore 116 near the bottom of the pump cylinder 112. The interior of the bottom of the pump cylinder 112 below the greater diameter bore 116, and also the area immediately between the greater diameter bore 116 and the main diameter bore 114, are both tapered to facilitate entry of the piston. The top of the main diameter bore 114 terminates in a frustoconcial smaller diameter aperture 118 leading to the top of the upper surface portion 102 of the cassette body 100. The smaller diameter aperture 118 is tapered, having the smaller diameter at the top.

Extending from the rear of the exterior of the bubble trap 104 and facing the pump cylinder 112 are two piston retaining fingers 120 and 122 (Figures 2 and 4), defining slots. The slots defined by the two piston retaining fingers 120 and 122 face each other,and are open at the bottom to accept in a sliding fashion a flat segment fitting between the two piston retaining fingers 120 and 122. The two piston retaining fingers 120 and 122 extend from the lower surface of the upper surface portion 102 of the cassette body 100 to a position between the bottom of the pump cylinder 112 and the bottom of the bubble trap 104.

Also extending from the bottom side of the upper surface portion 102 of the cassette body 100 are two latch supporting fingers 124 and 126 (Figures 1 to 4 and 7). The latch supporting finger 124 extends downwards from the left side of the upper surface portion 102 and at the bottom extends towards te right slightly to form an L-shape in cross section. The latch supporting finger 124 extends towards the front of the cassette body 100 further than the upper surface portion 102 does and terminates approximately two-thirds of the way towards the back of the upper surface portion 102 of the cassette body 100.

The latch supporting finger 126 extends downwards from the bottom of the upper surface portion 102 of the cassette body 100 with the left side of the bubble trap 104 forming a portion of the latch supporting finger 126. At the bottom, the latch supporting finger 126 extends towards the left slightly to form a backwards L-shape in cross section. The latch supporting finger 126 parallels the latch supporting finger 124, and is equal in depth (Figure 4). The latch supporting fingers 124 and 126 together will hold the slide latch, to be described below.

The passageways located in the top of the upper surface portion 102 of the cassette body 100 will now be described with primary reference to Figure 1. These passageways are all open on the top side of the upper surface portion 102, and are generally U-shaped as they are recessed into the top of the upper surface portion 102. A first passageway 128 communicates with the bore 110 in the siphon tube 108 of the bubble trap 104 at one end and extends towards the back of the upper surface portion 102 to a location to the right of the smaller diameter aperture 118 of the pump cylinder 112.

A cylindrical pressure plateau 130, which is essentially circular when viewed from the top, extends above the upper surface portion 102 slightly to the left of centre. The top of the pressure plateau 130 is flat, with a channel 132 extending across this flat top. The channel 132 extends from the five o'clock to eleven o'clock positions as viewed from the top in Figure 1, with the back of the cassette body being at 12 o'clock. The channel 132 is also shown in cross-section in Figure 115, and in a cutaway view in Figure 116. The depth of the channel 132 in the surface of the pressure plateau 130 is not quite as great as the height of the pressure plateau 130 above the upper surface portion 102 of the cassette body 100, with the channel 132 gradually becoming deeper with a smooth transition at the edges of the pressure plateau 130 to extend into the upper surface portion 102 of the cassette body 100 (Figure 116).

A second passageway 134 in the top of the upper surface portion 102 begins at a location to the left of the smaller diameter aperture 118 of the pump cylinder 112, and extends towards the front of the upper surface portion 102 approximately above the latch supporting finger 126. The second passageway 134 then travels to the left to connect in fluid communication with the end of the channel 132 located at the five o'clock position. A third passageway 136 in the top of the upper surface portion 102 begins at the end of the channel 132 loated at the eleven o'clock position and extends towards the back and left of the cassette body 100.

At the end of the third passageway 136, there is a recessed lens portion 138, which is used to focus and reflect light to detect air bubbles passing in front of it. The recessed lens portion 138 is also recessed into the top of the upper surface portion 102 of the cassette body 100 to allow fluid to pass therethrough. The recessed lens portion 138 is part of the apparatus which forms the subject of a co-pending patent application. A fourth passageway 140 in the top of the upper surface portion 102 begins at the other side of the recessed lens portion 138 from the third passageway 136, and extends from the left and back of the cassette body 100 towards the front and right around the pressure plateau 130 to a location at approximately seven o'clock on the pressure plateau 130. It should be noted that the fourth passageway 140 is spaced away from the pressure plateau 130 to allow for sealing means therebetween.

The end of the fourth passageway 140 terminates at the seven o'clock position relative to the pressure plateau 130 in an aperture 142 extending through the upper surface portion 102 (Figure 1). Located underneath the upper surface portion 102 concentrically around the aperture 142 is an outlet tube mounting cylinder 144 (Figures 3 an 4) which is in fluid communication with the aperture 142. The outlet tube mounting cylinder 144 extends downwards from the bottom of the upper surface portion 102 to a position above the portions of the latch supporting fingers 124 and 126 which extend parallel to the upper surface 102 of the cassette body 100. A support fin 145 extends to the right from the front of the outlet tube mounting cylinder 144.

Located on top of the upper surface 102 of the cassette body 100 is a slightly raised border 146 (Figures 1 and 2) which completely surrounds the first passageway 129, the smaller diameter aperture 118, the second passageway 134, the pressure plateau 130, the third passageway 136, the recessed lens portion 138, and the fourth passageway 140. The slightly raised border 146, which is used for sealing purposes, closely surrounds the edges of all these parts of the cassette body 100, except that it is spaced away from the portions of the first passageway 128 and the second passageway 134 adjacent the smaller diameter aperture 118, and the smaller diameter 118.

The form of the boarder 146 around the smaller diameter aperture 118 is generally rectangular with its longer sides located to the front and back and spaced away from the valve diaphragm 170, and its shorter sides to the right of the portion of the first passageway 129 adjacent the smaller diameter aperture 118 and to the left of the portion of the second passageway 134 adjacent the smaller diameter aperture 118. The rectangle is broken only at those locations where the first and second passageways 128, 134 extend towards the front of the cassette body 100.

The border 146 has a segment 147 located between the portion of the first passageway 128 adjacent the smaller diameter aperture 118 and the smaller diameter aperture 118 itself, with the segment 147 extending between the two longer sides of the rectangle. It also has another segment 149 located between the portion of the second passageway 134 adjacent the smaller diameter aperture 118 and the smaller diameter aperture 118 itself, with the segment 149 extending between the two longer sides of the rectangle. The border 146 is also spaced away from the sides of the pressure plateau 130, and the portions of the second passageway 134 and the third passageway 136 immediately adjacent the pressure plateau 130.

Located at the back of the upper surface 102 of the cassette body 100 are three cassette identifying indicia 148,150, and 152. The first and third cassette identifying indicia 148 and 152 are small, solid cylinders extending upwards from the top of the upper surface 102 (Figures 1 and 3).

The second cassette identifying indicia 150 is a prism cut into the under side of the upper surface 102 of the cassette body 100 (Figure 4). The first, second, and third indicia 148,150 and 152 are the subject of a co-pending patent application. It will be noted that the indicia 148,150 and 152 may be in any order or configuration, and are used for different ID codes to identify up to eight different cassettes. Additional ID bits could also be used if more than eight different cassettes were to be used. If redundant codes are desired, the three bits would of course accommodate the use of less than eight different cassettes.

Completing the construction of the cassette body 100 are five hollow cylinders 154, 156, 158, 160 and 162 protruding from the top surface of the upper surface 102 of the cassette body 100, an aperture 161 and a slot 164 located in the top of the upper surface 102, and a slot 166 located in the top surface of the latch supporting finger 124. Four of the hollow cylinders 154, 156, 158 and 160 are located around the pressure plateau 130, with the fifth hollow cylinder 162 being located to the left of the aperture 110 over the bubble trap 104. The aperture 161 is located in the top of the upper surface 102 in front and to the right of centre of the pressure plateau 130. The slot 164 is located in the top of the upper surface 102 near the back and the right hand side. The slot 166 is located in the top surface of the latch supporting finger 124 near the front of the cassette body 100.

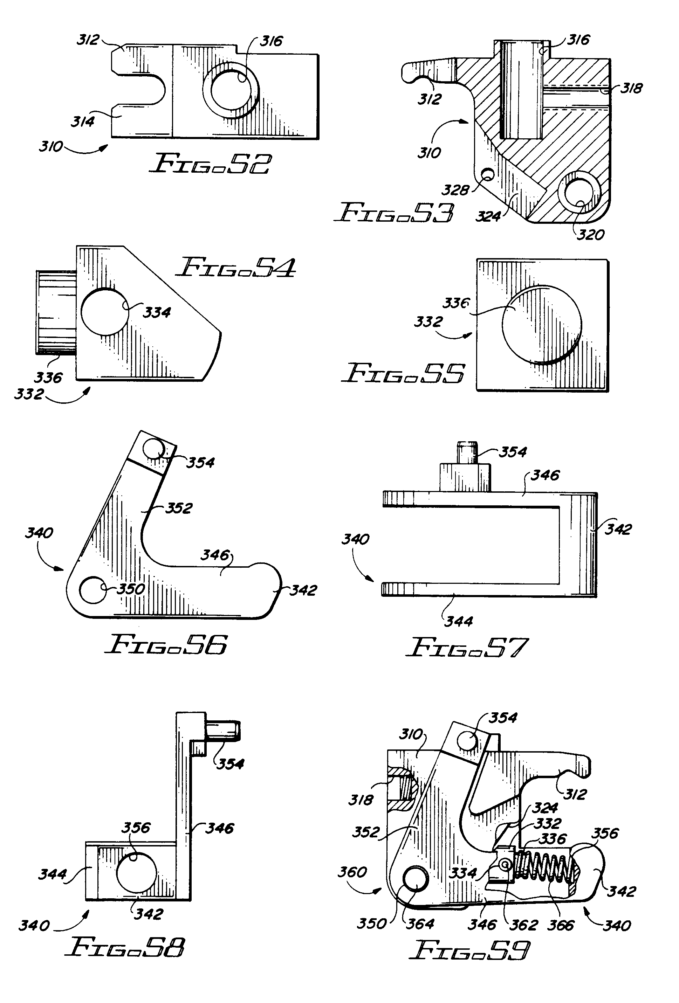

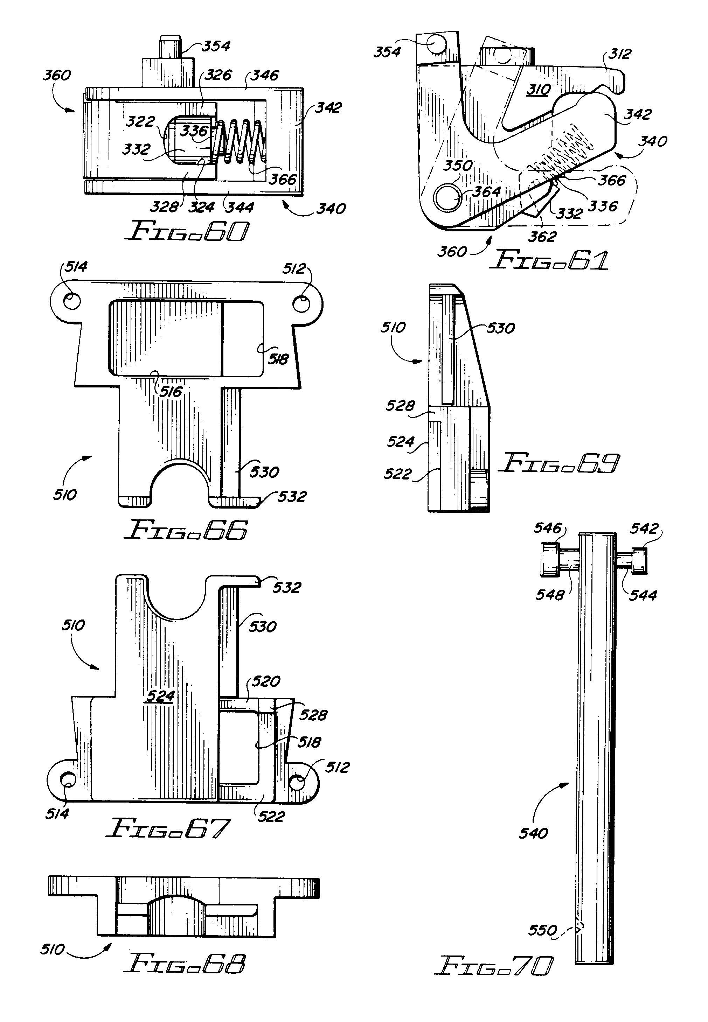

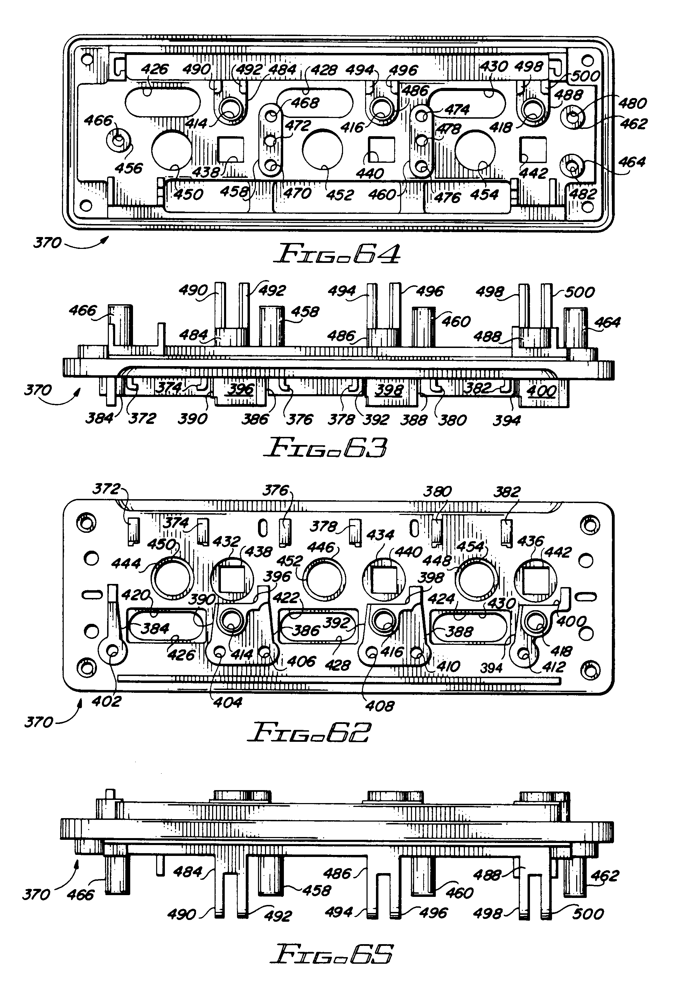

Referring now to Figures 9 to 12, a valve diaphragm 170 is shown which is arranged and configured to fit over the top of the upper surface 102 of the cassette body 100. The valve diaphragm 170 is made of flexible, resilient material, such as a medical grade silicone rubber. The hardness of the material used for the valve diaphragm 170 would be between thirty and fifty on the Shore A scale, with the preferred embodiment having a hardness of approximately thirty-five. The valve diaphragm 170 has three primary functions, the fist of which is to seal the tops of the first, second, third, and fourth passageways 128, 134, 136 and 140, respectively. Accordingly, the main surface of the valve diaphragm 170 is flat, and is sized to fit over the first, second, third, and fourth passageways 128, 134, 136 and 140, respectively, and also over the entire slightly raised border 146. The flat portion of the valve diaphragm 170 has three apertures 172, 174, and 176, and a notch 175 to accommodate the hollow cylinders 156, 160 and 62 and a pin fitting into the aperture 161 (Figure 1), respectively, and to align the valve diaphragm 170 in position over the top of the upper surface 102. It should be noted that the valve diaphragm 170 does not necessarily surround the other two hollow cylinders 154, 158.

The second primary function of the valve diaphragm 170 is to provide both an inlet valve between the first passageway 128 and the smallest diameter aperture 118 leading to the pump cylinder 112, and to provide an outlet valve between the smaller diameter aperture 118 and the second passageway 134. To fulfil this function the valve diaphragm 170 has an essentially rectangular domed portion 178 (Figures 9 to 12)forming a cavity 180 in the bottom of the valve diaphragm 170. When the valve diaphragm 170 is installed in position on the top of the upper surface 102 of the cassette body 100, the cavity 180 will be located just inside the rectangular portion of the slightly raised border 146 around the smaller diameter aperture 118 leading to the pump cylinder 112.

The cavity 180 will therefore be in fluid communication with the first passageway 128, the smaller diameter aperture 118, and the second passageway 134. Prior to installation of the cassette onto the main pump unit, the cavity 180 allows the open fluid path to facilitate priming of the cassette, where all air is removed from the system. Once primed, the cassette may be inserted onto the main pump unit and the cavity 180 will contact valve actuators to prevent free flow through the cassette. By using an inlet valve actuator to force the domed portion 178 onto the segment 147 of the slightly raised border 146, the flow of fluid between the first passageway 128 and the smaller diameter aperture 118 will be blocked, but flow between the smaller diameter aperture 118 and the second passageway 134 will be unaffected. Likewise, by using an outlet valve actuator to force the domed portion 178 onto the segment 149 of the slightly raised border 146, flow between the smaller diameter aperture 118 and the second passageway 134 will be blocked, but flow between the first passageway 128 and the smaller diameter aperture 118 will be unaffected. Extending around and spaced away from the front and sides of the domed portion 178 on the top surface of the valve diaphragm 170 is a U-shaped raised rib 181, the legs of which extend to the back of the valve diaphragm 170 (Figure 9).

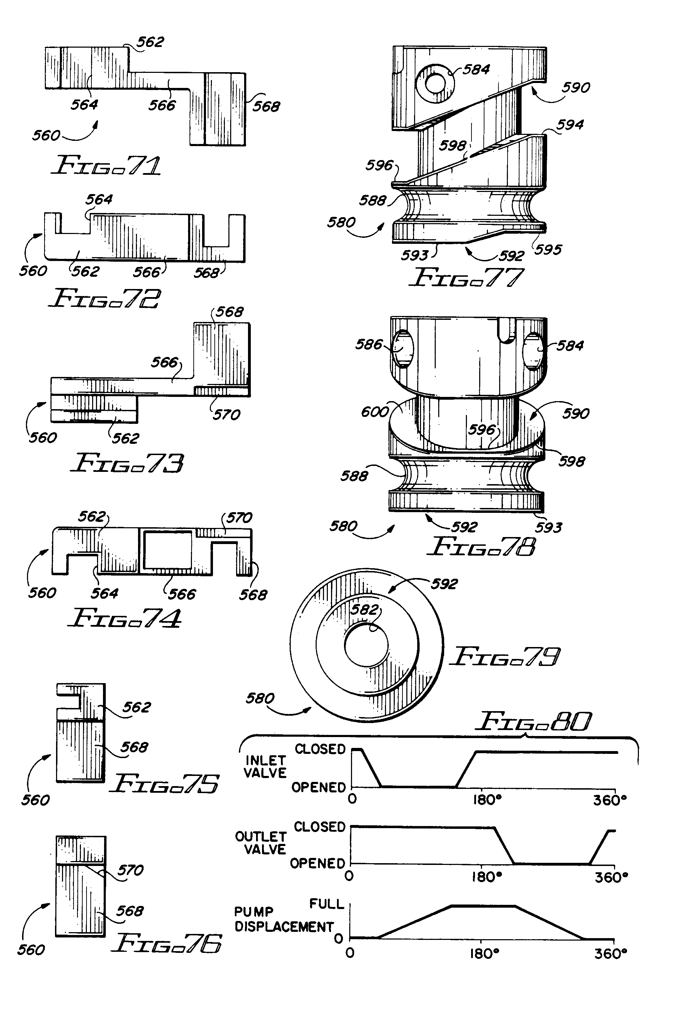

The third primary function of the valve diaphragm 170 is to provide a pressure diaphragm which may be used to monitor outlet fluid pressure. Accordingly, the valve diaphragm 170 has a pressure diaphragm 182 which is supported atop an upper cylindrical segment 184, which in turn is located on a lower cylindrical segment 186 extending above the surface of the valve diaphragm 170. The cylindrical segments 184 and 186 have the same inner diameter but the lower cylindrical segment 186 has a greater outer diameter. Thus, a portion of the top of the lower cylindrical segment 186 extends around the bottom of the upper cylindrical segment 184, creating a lip 188. In the preferred embodiment, the pressure diaphragm 182 may be domed slightly, as seen in Figure 11.

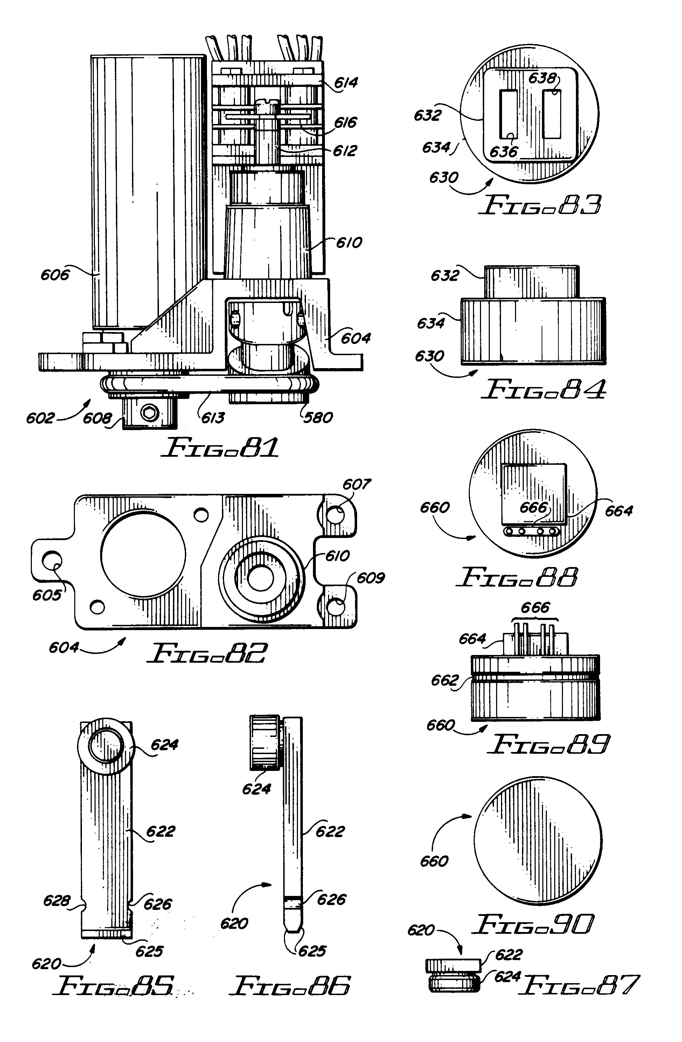

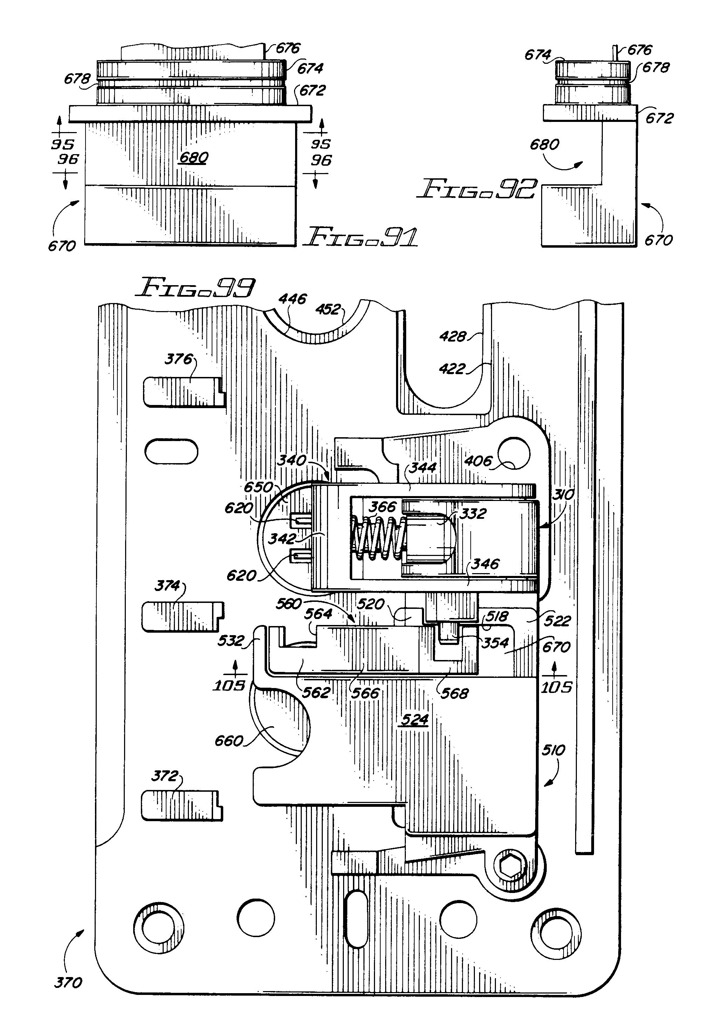

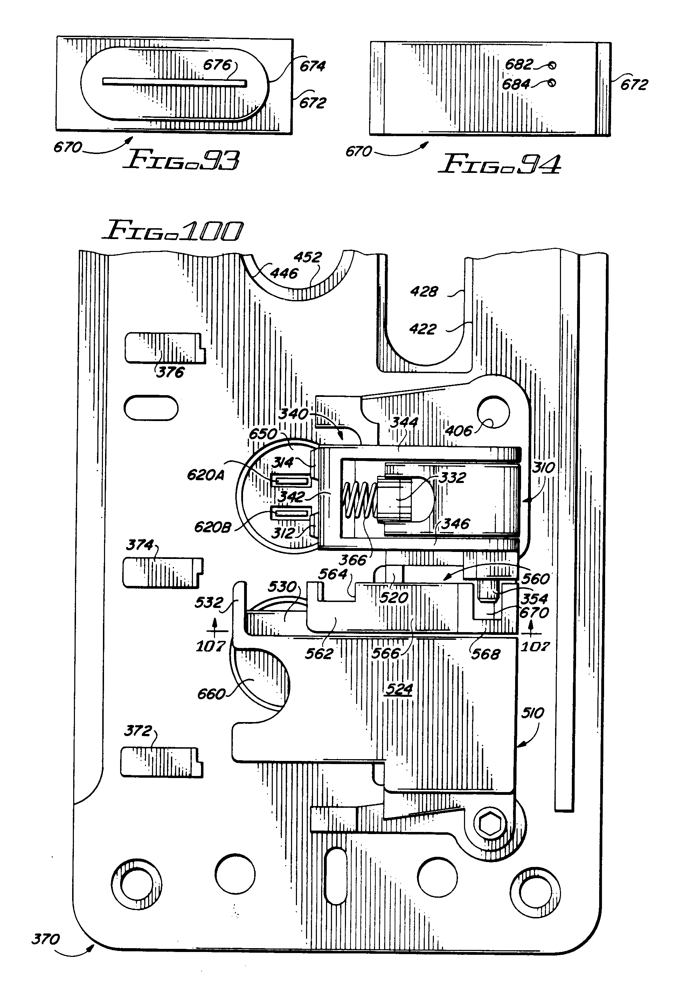

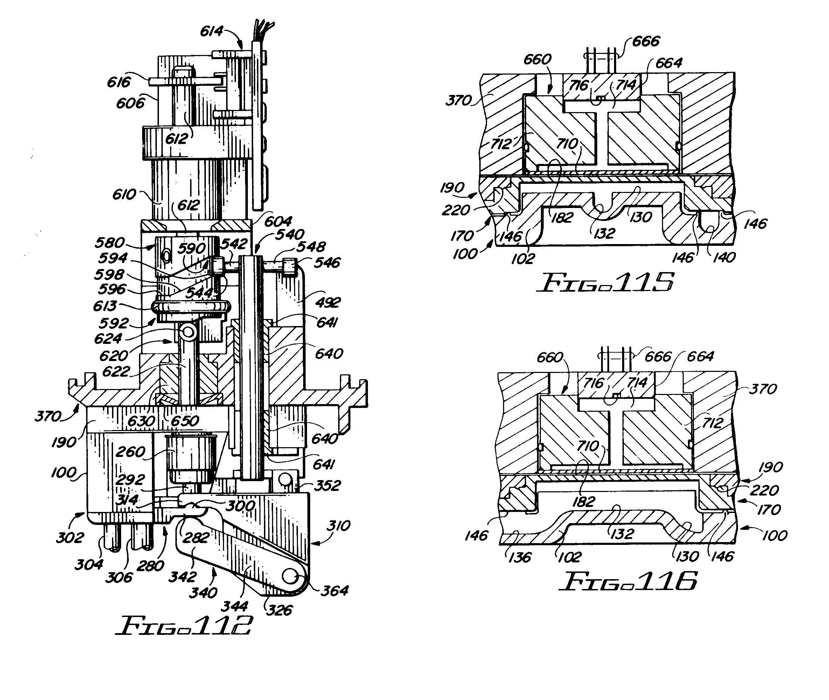

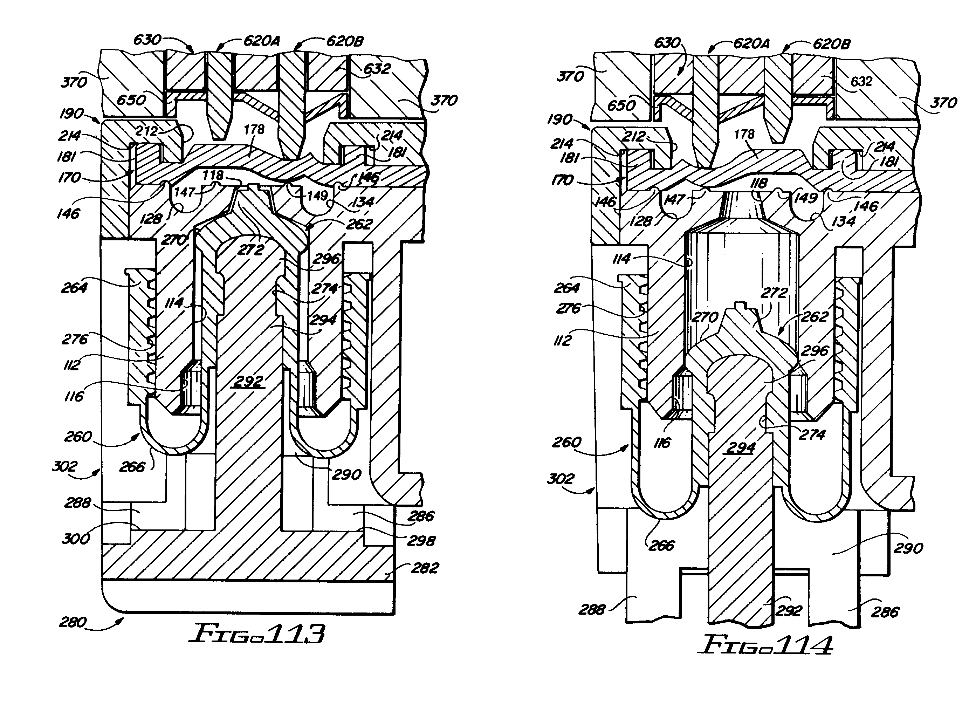

Turning now to Figures 13 to 23, a retainer cap 190 is shown which fits over the valve diaphragm 170 after it is mounted on the top of the upper surface 102 of the cassette body 100. The retainer cap 190 thus functions to cover the top of the cassette body 100, retaining the valve diaphragm 170 between the retainer cap 190 and the cassette body 100 in a sealing fashion. The retainer cap 190 thus has the same general outline when viewed from above (Figure 12) as the cassette body 100. Located in the bottom of the retainer cap 190 (Figure 14) are six pins 192, 194, 196, 198, 200, and 199, which are to be received by the hollow cylinders 154, 156, 158, 160 and 162 and the aperture 161, respectively, in the cassette body 100 to align the retainer cap 190 on the cassette body 100. Also located in the bottom of the retainer cap 190 is a tab 202 to be received by the slot 164, and tab 204 to be received by the slot 166.

The retainer cap 190 has three apertures 206, 208 and 210 therethrough located to coincide with the locations of the first, second and third cassette identifying indicia 148, 150 and 152. The size of the three apertures 206, 208 and 210 is sufficient to receive the small, solid cylinders which represent the first and third cassette idenfying indicia 148, 152.

Located in the retainer cap 190 is a rectangular aperture 212 (Figures 13, 14, 19 and 20) for location over the domed portion 178 on the valve diaphragm 170. The rectangular aperture 212 is slightly larger than the domed portion 178 to prevent any closure of the cavity 180 formed by the domed portion 178 when the retainer cap 190 is placed over the valve diaphragm 170 and the cassette body 100. The domed portion 178 of the valve diaphragm 170 will therefore protrude through the rectangular aperture 212 in the retainer cap 190. In the bottom of the retainer cap 190 around the rectangular aperture 212 is a U-shaped groove 214 (Figure 14) designed to accommodate the U-shaped raised rib 181 on the valve diaphragm 170.

Also located in the retainer cap 190 is a circular aperture 216 (figures 13 nd 14), which has a diameter slightly larger than the outer diameter of the upper cylindrical segment 184 on the valve diaphragm 170, to allow the upper cylindrical segment 184 and the pressure diaphragm 182 to protrude from the circular aperture 216 in the retainer cap 190. The diameter of the circular aperture 216 is smaller than the outer diameter of the lower cylindrical segment 186; on the underside of the retainer cap 190, disposed concentrically around the circular aperture 216, there is a cylindrical recess 218 arranged to receive the lower cylindrical segment 186. A circular raised bead 220 (Figures 14, 19 and 21) is located in the cylindrical recess 218 to help in the sealing of the cassette as it is assembled.

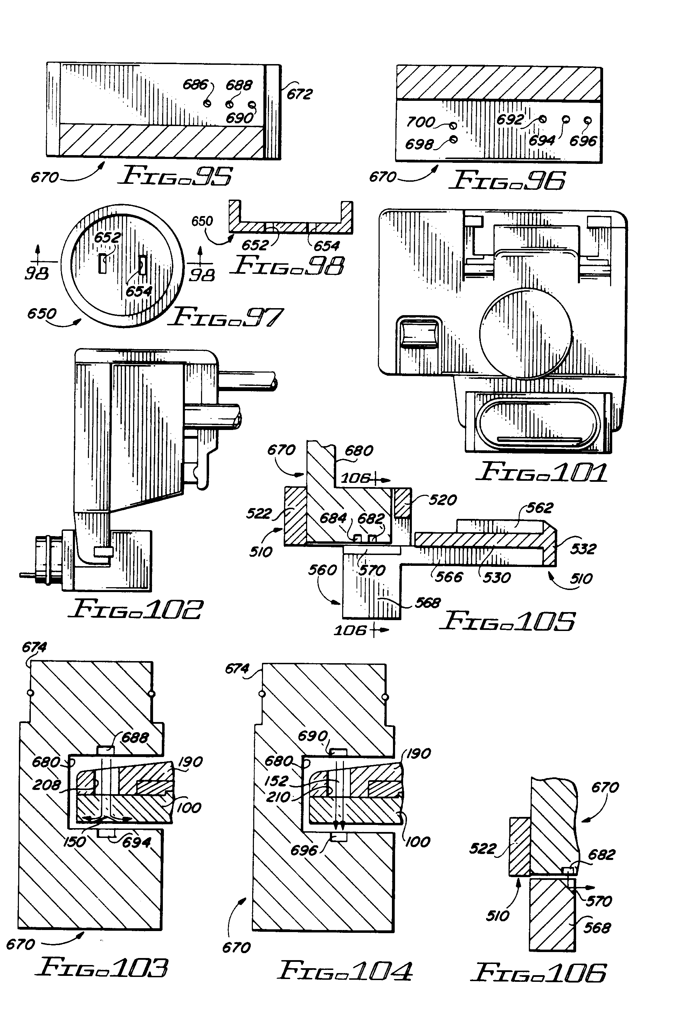

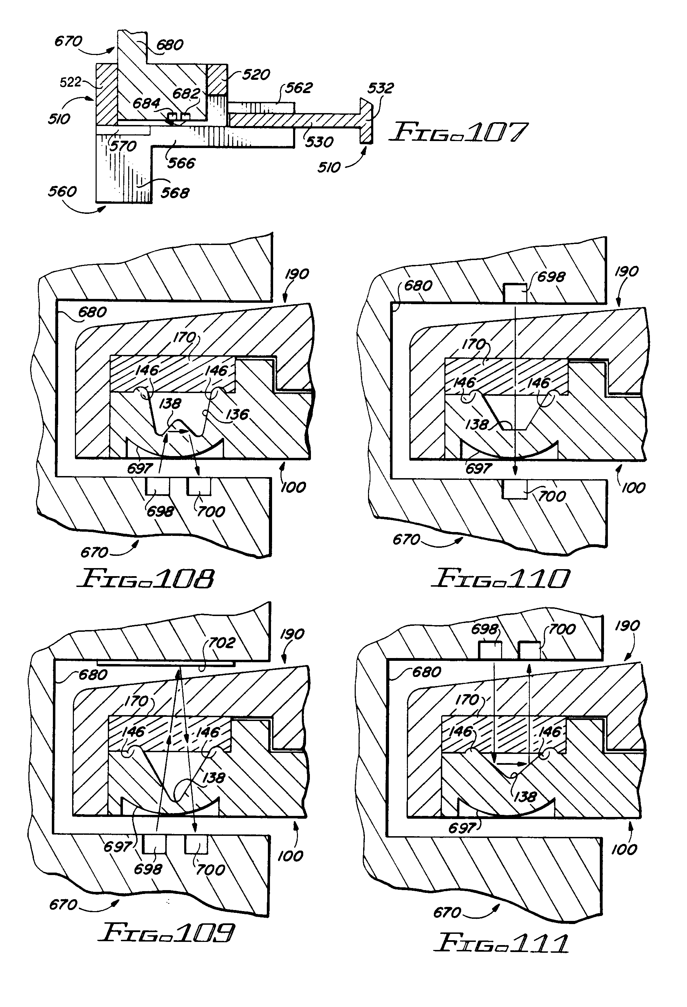

The retainer cap 190 has a front edge 222 (Figure 16), a back edge 224 (Figure 15) and left (Figure 18) and right (Figure 17) side edges 226 and 228 respectively. The edges 222, 224, 226 and 228 will contact the top of the upper surface 102 when the retainer cap 190 is assembled onto the cassette body 100 with the valve diaphragm 170 disposed therebetween. The retainer cap 190 is attached to the cassette body 100 in the preferred embodiment by ultrasonic welding, but adhesives or other bonding techniques known in the art may also be used.

Referring next to Figures 22 to 26, a bubble chamber cap 230 is illustrated which is placed over the open bottom of the bubble trap 104. The bottom of the cap 230 is the same size as the outer edge of the bottom of the bubble trap 104, and has a tab 232 (Figures 22 to 24) on the bottom which projects towards the back of the cassette beyond the back edge of the bubble trap 104. The cap 230 has a rectangular wall portion 234 extending upwards from the bottom defining a square space, the rectangular wall portion 234 being sized to fit inside the bubble chamber 106.

Located at the front and left sides of the rectangular wall portion 234 and extending upwards from the bottom of the bubble chamber cap 230, there is an inlet cylinder 236 having an inlet aperture 238. The inlet aperture 238 extends through the bottom of the bubble chamber cap 230 and is designed to receive a length of tubing from the bottom. The cap 230 is attached to the bottom of the bubble trap 104 in the cassette body 100 in the preferred embodiment by ultrasonic welding, but adhesives or other bonding techniques known in the art may also be used.

When the bubble chamber cap 230 is mounted on the bubble trap 104, the inlet cylinder 236 extends up to at least half of the height of the bubble chamber 106, and the siphon tube 108 draws fluid from the bottom of the siphon tube 108 in the space within the rectangular wall portion 234 of the bubble chamber cap 230. It will be appreciated by those skilled in the art that fluid will enter the bubble chamber 106 through the inlet aperture 238 in the inlet cylinder 236 near the top of the siphon tube 108, maintaining all air bubbles above the level near the bottom of the bubble chamber 106 at which fluid is drawn from the bubble chamber 106 by the siphon tube 108.

Figures 27 to 32 show a slide latch 240 which serves two main functions in the cassette. The slide latch 240 first serves to latch the cassette into place in a main pump unit. It also serves to block the flow of fluid through the cassette when it is not installed; closing the slide latch 240 to lock the cassette into place on the main pump unit also simultaneously allows the flow of fluid through the cassette. The slide latch 240 slides from the front of the cassette body 100 between the latch supporting fingers 124 and 126.

The slide latch 240 has an essentially rectangular, flat front portion 242 which is equal in height to the cassette body 100 with the retainer cap 190 and the bubble chamber cap 230 installed, and is equal in width to the distance between the left side of the bubble trap 104 and the left side of the cassette body 100. Two small notches 244 and 246 are removed from the rear of the front portion 242 at the top left and right corners respectively.

Extending from the rear of the front portion 242, about three-quarters of the way down, there is a horizontal bottom portion 248 which has its edges directly below the closest edges of the small notches 244 and 246. An inverted angled or L-shaped portion 250 extends from the inner edge of the small notch 244 at the top of the slide latch 240 down to the bottom portion 248. Similarly, an inverted, backwards angled or L-shaped portion 252 (Figures 27 and 28) extends from the inner edge of the small notch 246 at the top of the slide latch 240 down to the bottom portion 248.

Spaced outwardly from the left side of the bottom portion 248 and the left side of the leg of the L-shaped portion 250 is a left slide side 254. Likewise, spaced outwardly from the right side of the bottom portion 248 and the right side of the leg of the L-shaped portion 252 is a right slide side 256 (Figures 28 and 30). The left and right slide sides 254 and 256 are located slightly above the bottom of the bottom portion 248 and are of a suitable height for engagement in the latch supporting fingers 124 and 126 respectively.

An elongated tear-shaped aperture is located in the bottom portion 248, with the wider portion towards the front of the slide latch 240 and the extended narrower portion towards the back. When the slide latch 240 is inserted into the latch supporting fingers 124 and 126 on the cassette body 100, and the slide latch 240 is pushed fully towards the back of the cassette body 100, the wider portion of the aperture 258 will be aligned with the aperture 142 in the outlet tube mounting cylinder 144 (Figure 4) to allow a segment of tubing (not shown) leading from the aperture 142 to remain open. When the slide 240 is pulled out from the front of the cassette body 100, the segment of tubing (not sown) will be pinched off by the narrower portion of the aperture 258.

It is critical that the design and location of the elongate, tear-shaped aperture 258 ensure that the slide latch 240 engages the main pump unit before the tubing is opened and fluid is allowed to flow through the cassette. Likewise, the tubing must be pinched off and the fluid path through the cassette must be closed before the slide latch 240 releases the cassette from the main pump unit. In addition, the choice of material for the slide latch 240 is important, with a lubricated material allowing the pinching operating to occur without damaging the tubing (not shown). Examples of such materials are silicone or Teflon impregnated acetals such as Delren.

Located at the back of the slide latch 240 on the inside of the right slide side 256 at the bottom, there is a tab 257 (Figures 27, 30 and 32) which is used to engage the main pump unit with the cassette when the slide is closed. Located on the top side of the bottom portion 248 to the right of the aperture 258 is a small wedge-shaped retaining tab 259 (Figure 27, 30 and 32). The retaining tab 259 cooperates with the bottom of the support fin 145 to resist the slide latch 240 from being freely removed once installed into the cassette body 100. When the slide latch 240 is pulled back out from the front of the cassette body 100 so that the wider portion of the aperture 258 is aligned with the aperture 142 in the outlet tube mounting cylinder 144, the retaining tab 259 will engage the slightly raised border 146, resisting the slide latch 240 from being drawn further out.

Referring now to Figures 33 to 36, a one-piece piston cap and boot seal 260 is illustrated, which is for use on and in the pump cylinder 112 (Figures 3 and 8). The piston cap and boot seal 260 is of a one-piece construction, and is made of flexible, resilient material, such as silastic (silicone rubber) or medical grate natural rubber. Natural rubber may be used to minimise friction, since some sticking of the a silicone rubber piston cap and boot seal 260 in the pump cylinder 112 may tend to occur. Teflon impregnated silastic or some other proprietary formula widely available would overcome this problem. In addition, the piston cap and boot seal 260 may be lubricated with silicone oil prior to installation in the pump cylinder 112. The advantage of using silastic is that it may be radiation sterilised, whereas natural rubber must be sterilised using a gas such as ethylene oxide. in addition, silastic has better wear characteristics than natural rubber, making it the preferred choice.

The piston cap and boot seal 260 includes a piston cap portion indicated generally at 262, and a bottom seal portion comprising a retaining skirt 264 and a thin rolling seal 266. The piston cap portion 262 includes a hollow cylindrical segment 268 having an enlarged, rounded piston cap head 270 at the top. The piston cap head 270 has a roughly part-elliptical cross-section, and the outer diameter of the sides provides a dynamic seal in the main diameter bore 114 of the pump cylinder 112. The roughly elliptical configuration of the piston cap head 170 closely fits the top of the main diameter bore 114 of the pump cylinder 112. Extending from the top of the piston cap head 270 at the centre is a frustoconical segment 272, with the larger diameter at the bottom. The frustoconical segment 272 fits closely in the smaller diameter aperture 118 of the pump cylinder 112.

The hollow cylindrical segment 268 and the piston cap head 270 together define a closed end to the piston cap and boot seal 260, which receives a piston as will described below. The hollow cylindrical segment 268 has a smaller diameter portion 274, which is spaced away from the end of the piston cap head 270 to provide retaining means to retain a piston in the segment 268 between the piston cap head 270 and the smaller diameter portion 274.

The retaining skirt 264 is essentially cylindrical, and is designed to fit snugly around the outer diameter of the pump cylinder 112. Prior to installation and with the piston cap and boot seal 260 in a relaxed configuration as shown in Figures 33 to 36, the retaining skirt 264 is located roughly around the hollow cylindrical segment 268. The retaining skirt 264 has an internal diameter sufficiently small to retain it in position around the pump cylinder 112 without moving when the piston cap portion 262 moves.

Located around the inner diameter of the retaining skirt 264 is a tortuous path 276 leading from one end of the retaining skirt 264 to the other. The tortuous path 276 is required for sterilisation of the assembled cassette, allowing the sterilising gas to sterilise the area between the inside of the pump cylinder 112 and the piston cap and boot seal 260, which would be closed and could remain unsterilised if the tortuous path 276 were not present. In addition, since the sterilising gas is hot and cooling occurs rapidly after the sterilising operation, the tortuous path 276 allows pressure equalisation to occur rapidly where it otherwise would not. In the preferred embodiment, the tortuous path 276 is a series of threads in the inner diameter of the retaining skirt 264.

Completing the construction of the piston cap and boot seal 260 is the rolling seal 266, which is a segment defined rotating about the axis of the piston cap and boot seal 260, a U having a first leg at the radius of the hollow cylindrical segment 268 and a second leg at the radius of the retaining skirt 264, with the top of the first leg of the U being attached to the bottom of the hollow cylindrical segment 268 and the top of the second leg of the U being attached to the bottom of the retaining skirt 264. When the piston cap and boot seal 160 is installed and the piston cap portion 262 moves in and out in the main diameter bore 114 in the pump cylinder 112, the legs of the U will vary in length, with one leg becoming shorter as the other leg becomes longer. In this matter, the rolling seal 266 provides exactly what its name implies - a seal between the piston cap portion 262 and the retaining skirt 264 which rolls as the piston cap portion 262 moves.

Figures 37 to 42 show a piston assembly 280 which drives the piston cap portion 262 of the piston cap and boot seal 260 in the pump cylinder 112. The piston assembly 280 has a horizontal rectangular base 282 located directly behind the bubble chamber cap 230 when the piston cap portion 262 is fully inserted into the pump cylinder 112. The rectangular base 282 has a notch 284 in its front edge which is slightly larger than the tab 232 in the bubble chamber cap 230 (Figure 23).

Extending upwards from the front edge of the rectangular base 282 to the left of the notch 284 is an arm 286, and to the right side of the notch 284, an arm 288. At the top of the arms 286 and 288 there is a vertically extending rectangular portion 290. The rectangular portion 290 and the upper portions of the arms 286 and 288 are for insertion between the piston retaining fingers 120 and 122 in the cassette body 100 (Figure 4).

The top of the rectangular portion 290 will contact the bottom of the upper surface 102 of the cassette body 100 to limit upward movement of the piston assembly 280, the rectangular base 282 being approximately even with the bubble chamber cap 230 installed in the bottom of the bubble trap 104 of the cassette body 100 when the piston assembly 280 is in its fully upward position. The bottom of the rectangular portion 290 will contact the tab 232 on the bubble chamber cap 230 when the piston assembly 280, the piston head 296, and the piston cap portion 262 are fully retracted from the pump cylinder 112.

A cylindrical piston rod 292 extends upwards from the rectangular base 282 at a central position near the back edge. At the top of the piston rod 292 there is a reduced diameter cylindrical portion 294, and above this a cylindrical piston head 296. The diameter of the piston head 296 is larger than the diameter of the reduced diameter cylindrical portion 294, and the top of the piston head 296 has rounded edges in the preferred embodiment. The piston head 296 is designed to be received in the portion of the hollow cylindrical segment 268 between the smaller diameter portion 274 and the piston cap head 270 in the piston cap portion 262 (Figure 36). The reduced diameter cylindrical portion 294 is designed to be received in the smaller diameter portion 274 of the piston cap portion 262.

The top of the piston head 296 is slightly above the top of the rectangular portion 290, and when the piston assembly 280 is in its fully upward position, the piston head 196 will have brought the piston cap head 270 and the frustoconical segment 272 thereon (Figure 36) to the top of the pump cylinder 112 and into the smaller diameter aperture 118 (Figure 8), respectively, to eliminate completely any volume within the pump cylinder 112 and within the smaller diameter aperture 118.

Two raised beads 298 and 300, complete the construction of the piston assembly 280 with the raised bead 298 being on the top surface of the rectangular base 282 on the left side of the piston rod 292, and the raised bead 300 being similarly located on the right. Both of the raised beads 298 and 300 extend from the piston rod 292 laterally towards the sides of the rectangular base 282. The raised beads 298 and 300 will be used to centre the piston assembly 280 relative to the jaws of the main pump unit used to drive the piston assembly 280, and will also serve to retain the piston assembly 180 in the jaws.

The assembly and configuration of the cassette will now be described with reference to the assembled cassette 302 shown in Figure 43 to 48, and with reference to other figures specifically mentioned in the discussion. The valve diaphragm 170 is placed over the top of the upper surface 102 of the cassette body 100, with the apertures 172, 174, and 176 placed over the hollow cylinders 156, 160, and 162 respectively. the retainer cap 190 is then located over the valve diaphragm 170 and the cassette body 100, and is secured in place by ultrasonic welding. While adhesive sealing may be used, it is more difficult to ensure the consistent hermetic seal required in the construction of cassette 302.

The step of firmly mounting the retainer cap 190 onto the cassette body 100 exerts a bias on the valve diaphragm 170 (Figure 9) causing it to be compressed in certain areas, particularly over the slightly raised border 146 on the upper surface 102 of the cassette body 1000. This results in excellent sealing characteristics, and encloses the various passageways located in the upper surface 102 of the cassette body 100. The first passageway 128 is enclosed by the valve diaphragm 170, communicating at one end with the aperture 110 and at the other end with the area between the cavity 180 and the upper surface 102 of the cassette body 100. The second passageway 134 also communicates with the area between the cavity 180 and the upper surface 102 of the cassette body 100 at one end with its other communicating with one end of the passageway 132 in the pressure plateau 130.

The pressure diaphragm 182 is located above the surface of the pressure plateau 130 (Figures 115 and 116), leaving a space between the edges at the side of the pressure plateau 130 and the inner diameters of the upper cylindrical segment 184 and the lower cylindrical segment 186. This allows the pressure diaphragm 182 to be quite flexible, a design feature essential to the proper operation of the pressure monitoring apparatus. It may therefore be appreciated that the flow area between the second passageway 134 and the third passageway 136 is not just the area of the passageway 132, but also the area between the pressure diaphragm 182 and the pressure plateau 130, as well as the area around the sides of the pressure plateau 130 adjacent the upper cylindrical segment 184 and the lower cylindrical segment 186.

The third passageway 136 (Figure 1) is also included by the valve diaphragm 170 (Figure 9), and communicates at one end with the passageway 132, and at the other end with the recessed lens portion 138. The fourth passageway 140 is enclosed by the valve diaphragm 170, and communicates at one end with the recessed lens portion 138 and at the other end with the aperture 142.

Next, the bubble chamber cap 230 is placed on the bottom of the bubble chamber 106, as shown in Figure 44, and is secured by ultrasonically sealing to the cassette body 100. The piston cap portion 262 of the piston cap and boot seal 260 (Figure 36) is inserted into the main diameter bore 114 of the pump cylinder 112 and pushed towards the top of the main diameter bore 114. Simultaneously, the retaining skirt 264 is located over the outside of the pump cylinder 112 and is moved up its outer surface to the position shown in Figures 46 to 48, which is nearly to the top of the outer surface of the pump cylinder 112. Next, the piston head 296 of the piston assembly 280 (Figures 37 and 40) is inserted into the hollow cylindrical segment 268 of the piston cap and boot seal 260, and is forced past the smaller diameter portion 274 until it snaps home, resting against the underside of the piston cap head 270.

The slide latch 240 is then inserted into engagement with the cassette body 100, which is accomplished by sliding the left slide side 254 into the latch supporting finger 124 (to its right) and by sliding the right slide side 256 into the latch supporting finger 126 (to its left). The slide latch 240 is then pushed fully home to align the wider portion of the elongate, tear-shaped aperture 258 with the outlet tube mounting cylinder 144. An inlet tube 304 is adhesively secured to the inner diameter of the inlet aperture 238 in the bubble chamber cap 230, in fluid communication with the bubble chamber 106. An outlet tube 306, extending through the wider portion of the aperture 258 and is adhesively secured to the inner diameter of the outlet tube mounting cylinder 144 in the cassette body 100, in fluid communication with the fourth passageway 140 through the aperture 142.

The inlet tube 304 and the outlet tube 306 are shown in the figures only in part; on their respective ends not connected to the assembled cassette 302 they may have connector fittings such as standard luer connectors (not shown), which are well known in the art. The adhesives used to attach the inlet tube 304 and the outlet tube 306 to the assembled cassette 302 also represent technology well known in the art. For example, adhesives such as cyclohexanone, methylene dichloride, or tetrahydrofuron (THF) may be used.

The Main Pump Unit The preferred embodiment of the main pump unit of the present invention includes a number of components used to hold, latch, and drive the cassette 302 described above. Referring first to Figures 49 to 53, a latch head 310 is illustrated which is used on grasp the raised beads 298 and 300 on the piston assembly 280 (Figure 37). Extending from the front of the latch head 310 at the top there is a left jaw 312 and a right jaw 314. The left and right jaws 312 and 314 have curved indentations on their under sides to receive the raised beads 298 and 300 (Figure 37), respectively. A space between the left jaw 312 and the right jaw 314 allows them to accommodate the piston rod 292.

A cylindrical aperture 316 is located in the top of the latch head 310, arranged to receive a shaft on which the latch head 310 is mounted. A threaded aperture 318 in the back side of the latch head 310 communicates with the cylindrical aperture 316, and will receive locking means to lock a shaft in the cylindrical aperture 316. An aperture 320 extends through the latch head 310 from left to right near the bottom at the rear.

A notch 322 is formed in the front of the latch head 310 at the bottom in the centre, leaving a left side portion 324 and a right side portion 326.

Aligned apertures 328 and 330 are formed in the side portions 324 and 326 respectively. In addition, the portion of the latch head 310 including the left jaw 312 has a raised edge 327 facing upwards and backwards, and a raised edge 329 facing downwards and forwards. The portion of the latch head 310 including the right jaw 314 has a raised edge 331 facing downwards and forwards. The raised edges 327, 329 and 331 will be used to limit the movement of the latch jaw, as will be described below.

A spring seat 332 is shown in Figures 54 and 55, which is designed to fit in the notch 322 in the latch head 310. The spring seat 332 has an aperture 334 extending through it from left to right, which is slightly larger than the apertures 328 and 330 on the latch head 310. The spring seat 332 also has a cylindrical segment 336 extending from the front.

Figures 56 to 58 show a latch jaw 340 which grasps the bottom of the rectangular base 282 of the piston assembly 280 (Figure 37) and maintains the left and right jaws 312 and 314 of the latch head 310 (Figure 51) in contact with the raised beads 298 and 300. The latch jaw 240 has a front jaw portion 342 approximately as wide as the left and right jaws 312 and 314 of the latch head 310. The jaw portion 342 contacts the bottom of the rectangular base 282 of the piston assembly 280.

Extending back from the jaw portion 342 is a left arm 344 and a right arm 346. The left arm 344 has an aperture 348 (not shown) away from the jaw portion 342 while the right arm 346 has an aperture 350 away from the jaw portion 342. The apertures 348 and 350 are slightly smaller in diameter than the aperture 320 in the latch head 310 (Figures 49 and 50).

Extending upwards from the end of the right arm 346 away from the jaw portion 342 at approximately sixty degrees is a driving arm 352. At the end of the driving arm 352 which is not attached to the right arm 346 is a link pin 354, extending to the right. Completing the construction of the latch jaw 240 is a cylindrical recess 356 in the rear side of the jaw portion 342, which has an inner diameter larger than the outer diameter of the cylindrical segment 336 of the spring seat 332 (Figure 55).

Referring now to Figures 59 to 61, the construction of jaws assembly 360 from the latch head 310, the spring seat 332, and the latch jaw 340 is illustrated. The spring seat 332 fits within the notch 322 and between the left and right jaws 312, 314 of the latch head 310. A pin 362 is inserted through the aperture 328 in the side portion 324, the aperture 334 in the spring seat 332, and the aperture 330 in the side portion 326. The pin 362 is sized to fit snugly in the apertures 328 and 330, thereby retaining the pin 362 in place and allowing the spring seat 332 to rotate about the pin 362.

The latch jaw 340 is mounted onto the latch head 310 with the left and right jaws 312 314 of the latch head 310 facing the jaw portion 342 of the latch jaw 340 using a pin 364. The pin 364 is inserted through the aperture 320 in the latch head 310 and the aperture 350 in the right arm 346. The pin 364 is sized to fit snugly in the apertures 348 and 350, thereby retaining the pin 354 in place and allowing the latch jaw 340 to rotate about the pin 364.

A spring 366 is located with one end mounted over the cylindrical segment 336 on the spring seat 332 and the other end mounted in the cylindrical recess 356 in the latch jaw 340. The spring 366 acts to bias the latch jaw 340 in either the open position shown in Figure 59 with the jaw portion 342 away from the jaws 312 and 314 of the latch head 310, or in the closed position shown in Figure 61, with the jaw portion 342 of the latch jaw 340 urged closely adjacent the jaws 312 and 314. The movement of the latch jaw 340 in both directions with respect to the latch head 310 is limited; to the position shown in Figure 59 by the driving arm 352 contacting the raised edge 327, and to the position shown in Figure 61 by the right arm 346 contacting the raised edge 329 and by the left arm 344 contacting the raised edge 331.

When the assembled cassette 302 is installed, movement of the latch jaw 340 to the position of Figure 61 will also be limited by the presence of the piston assembly 280, with the rectangular base 282 being grasped by the jaws assembly 360. It will be noted that by moving the pin 354 either toward the front or toward the back, the latch jaw 340 may either be opened or closed, respectively.

In Figures 62 to 65, a main pump unit chassis 370 is illustrated which is designed to mount three independent pump units including three drive mechanisms into which three disposable assembled cassettes 302 may be installed. The assembled cassettes 302 are mounted on the bottom side of the pump chassis 370 shown in Figure 62, with the motors and drive train being installed mounted on top of the pump chassis 370 (Figure 64) within a housing (not shown).

Located on the pump chassis 370 are three pairs of angled segment 372 and 374, 376 and 378 and 380 and 382. Each pair of angled segments 372/374, 376/378, 380/382 defines two facing channels between them. In the preferred embodiment, the angled segments are angled slightly further from the bottom of the pump chassis 370 near the front, to have a camming effect as an assembled cassette 302 is installed and the slide latch 240 is closed. Specifically, the angled segment 372 defines a channel facing the angled segment 374 and the angled segment 374 defines a channel facing the angled segment 372. The angled segment 376 defines a channel facing the angled segment 378, and the angled segment 378 defines a channel facing the angled segment 76. Finally, the angled segment 380 defines a channel facing the angled segment 382 and the angled segment 382 defines a channel facing the angled segment 380.

Each pair of angled segments 372/374, 376/378, 380/382, provides means on the bottom of pump chassis 370 for one assembled cassette 302 to be securely latched to. The inverted L-shaped portions 250 and 252 in the slide latch 240 (Figures 29 and 30) of the assembled cassette 302 can be attached to one of the paris of angled segments 372/374, 376/378 and 380/382. With the slide latch 240 pulled back away from the front of the assembled cassette 302, an area between the front portion 242 of the slide latch 240, and the top front of the cassette body 100 and retainer cap 190 is opened, allowing the top of the assembled cassette 302 to be placed over one of the pairs of angled segments 372/374, 376/378, 380/382.

By way of example, it will be assumed that the assembled cassette 302 is to be mounted in the first position ( the position on the left end of the pump chassis 370) on the first pair of angled segments 372/374. The top surface of the assembled cassette 302, which is the retainer cap 190 (Figure 43), will come into contact with the bottom of the pump chassis 370 (Figure 62). In order to allow the assembled cassette 302 to be installed, the slide latch 240 is pulled back fully from the front of the assembled cassette 302, leaving the open area between the front portion 242 of the slide latch 240 and the front top portion of the assembled cassette 302 (made up of the the cassette body 100 and the retainer cap 190) facing the front portion 242 of the slide latch 240.

The top of the assembled cassette 302 is then placed against the bottom of the pump chassis 370 with the first pair of angled segments 372/374 fitting in the area between the front portion 242 of the slide latch 240 and the front top portion of the assembled cassette 302. The slide latch 240 is then pushed forward into the cassette body 100, sliding the inverted L-shaped portion 250 of the slide latch 240 into engagement with the angled segment 372, and sliding the inverted, backwards L-shaped potion 252 of the slide latch 240 into engagement with the angled segment 374. The assembled cassette 302 will thus be held in position on the bottom of the pump chassis 370 until the slide latch 240 is again pulled back, releasing the assembled cassette 302.

Projecting from the bottom of the pump chassis 370 are a number of components used to position and align the assembled cassettes 302 in the first (left hand end of the pump chasses 370), second (intermediate), and third (right hand end) positions on the pump chassis 370. Three left lateral support walls 384, 386 and 388 protrude from the bottom of the pump chassis 370 at locations to support the rear upper left side portion of assembled cassettes 302 in proper positions in the first, second and third positions, respectively. Likewise, three right lateral support walls 390, 392 and 394 protrude from the bottom of the pump chassis 370 at locations to support the rear upper right portion of assembled cassettes 302 in proper positions in the first, second, and third positions, respectively.

Additional support and positioning for the installation of the assembled cassettes 302 into the first, second and third positions are provided for the upper right rear corner of the assembled cassettes 302 by three right corner support walls 396, 398, and 400 respectively. The three right corner support walls 396, 398, 400 are L-shaped when viewed from beneath (Figure 62), and support and position the rear of the assembled cassettes 302 behind the pump cylinders 112 (Figure 4) and a portion of the right side of the assembled cassettes 302 adjacent the pump cylinders 112. Note that the three right lateral support walls 390, 392, and 394 and the three right corner support walls 396, 398 and 400 together provide continuous support and positioning for the assembled cassettes 302 in the first, second and third positions, respectively.

A threaded aperture 402 is formed in the raised material forming the left lateral support wall 384, near the rear. A single segment of raised material forms the right lateral support wall 390, the right corner support wall 396, and the left lateral support wall 386. Formed in that segment of raised material near the rear is a threaded aperture 404 on the left side near the right lateral support wall 390, and a threaded aperture 406 on the right side near the left lateral support wall 386. Similarly, a single segment of raised material forms the right lateral support wall 392, the right corner support wall 398, and the left lateral support wall 388, and includes corresponding apertures 408 and 410 on the left and right respectively. Finally, a single segment of raised material forms the right lateral support wall 394 and the right corner support wall 400 and includes a threaded aperture 412 at the rear near the right lateral support wall 394.

In the segment of raised material forming the support walls 390, 396 and 386, near the former where the right lateral support wall 390 and the right corner support wall 396 meet, there is an aperture 414 which extends through the thickness of the pump chassis 370. A similar aperture 416 is formed in the segment of raised material forming the support walls 392, and 388. In the segment of raised material forming he right lateral and corner support walls 394 and 400 near the corner where these walls meet, there is an aperture 418 which extends through the thickness of the pump chassis 370.

When an assembled cassette 302 is positioned and mounted in the first, second, and third positions, the apertures 414, 416 and 418 respectively, will coincide directly with the piston rods 292 of the assembled cassettes 302 (Figure 46). The apertures 414, 416 and 418 will be used to mount the drive shafts connected to the jaws assembles 360 (Figures 59 to 61) used to drive the piston assembly 280.

Located between the left lateral support wall 384 and the right lateral support wall 390, there is a longitudinal rectangular recess 420 in the bottom surface of the pump chassis 370. Similarly, located between the left lateral support wall 386 and the right lateral support wall 392, there is a longitudinal rectangular recess 422 and located between the left lateral support wall 384 and the right lateral support wall 390, there is a longitudinal rectangular recess 424. While the rectangular recesses 420, 422, 424, do not extend through the pump chassis 370, oval apertures 426, 428, and 430 smaller than the rectangular recesses 420, 422, 424, are located in the rectangular recesses 420, 422, and 424 respectively, and extend through to the top side of the pump chassis 370. The rectangular recess 420, 422 and 424 will house sensor modules, and the oval aperture 426, 428 and 430 allow wires from the sensor modules to extend through the pump chassis 370. With the assembled cassettes 302 positioned and mounted in the fist, second, and third positions, the rear-most parts of the upper portions of the assembled cassettes 302 will be located beneath the rectangular recesses 420, 422 and 424.

Located in front of the right corner support wall 396 is a circular recess 432 in the bottom surface of the pump chassis 370.

Similar circular recesses 434 and 436 are formed in the bottom surface of the pump chassis 370 in front of the right corner support walls 398 and 400. While the circular recesses 432, 434, and 436 do not extend through the pump chassis 370, square apertures 438, 440 and 442, smaller than the circular recesses 432, 434, and 436, are located in the circular recesses 432, 434, and 436 respectively and extend through the top side of the pump chassis 370.

The circular recesses 432, 434, and 436 will be used to house valve actuator guides, and the square apertures 438, 440 and 442 allow valve actuators to extend through the pump chassis 370 and to orient the valve actuator guides. With the assembled cassettes 302 positioned and mounted in the first, second and third positions, the circular recesses 432, 434, and 436, respectively, will correspond exactly with the locations of the domed portions 178 of the valve diaphragms 170 in the assembled cassettes 302 (Figure 43).

Located to the left of the circular recess 432 and in front of the rectangular recess 420, there is a circular recess 444 in the bottom surface of the pump chassis 370. Similarly, located to the left of the circular recess 434 and in front of the rectangular recess 422, there is a circular recess 446, and located to the left of the circular recess 436 and in front of the rectangular recess 424, there is a circular recess 448. While the circular recesses 444, 446, 448 do not extend through the pump chassis 370, cylindrical apertures 450, 452 and 454 of a smaller diameter than the circular recesses 444, 446 and 448 are located in the circular recesses 444, 446 and 448 respectively, and extend through to the top side of the pump chassis 370.

The circular recesses 444, 446 and 448 will be used to house pressure transducers and the cylindrical apertures 450, 452, and 454 allow wires from the pressure transducers to extend through the pump chassis 370. With the assembled cassettes 302 positioned and mounted in the first, second, and third positions, the circular recesses 444, 446 and 448 respectively, will correspond with the locations of the pressure diaphragms 182 of the valve diaphragms 170 in the assembled cassettes 302 (Figure 43).

Projecting from the top surface of the pump chassis 370 are a number of raised elements in which threaded apertures are located to support the drive assembly. A cylindrical raised segment 456 is located to the left of the cylindrical aperture 450, a laterally extending generally oval raised segment 458 is located between the square aperture 438 and the cylindrical aperture 452, and a second laterally extending generally oval raised segment 460 is located between the square aperture 440 and the cylindrical aperture 454. In addition, two cylindrical raised segments 462 and 464 are located to the right of the square aperture 442 laterally aligned with the rear-most and front-most portions respectively of the oval raised segments 458 and 460.

A threaded aperture 466 is formed in the cylindrical raised segment 456. Located in the oval raised segment 458, there is a threaded aperture 468 near the rear, a threaded aperture 470 near the front, and a central threaded aperture 472. Similarly, the oval raised segment 460 includes a threaded aperture 474 near the ear, a threaded aperture 476 near the front, and a central threaded aperture 478. The cylindrical raised segments 462 and 464 include threaded apertures 480 and 482 respectively.

The apertures 414 and 416 and 418 through the pump chassis 370 terminate in raised segments 484, 486 and 488 respectively extending from the top surface of the pump chassis 370 and surrounding the respective apertures. Extending upwards from the raised segment 484 behind the aperture 414 on the left side is a guide finger 490, and on the right side is a guide finger 492. The guide fingers 490 and 492 are parallel and have a space between them. Similar pairs of guide fingers 494/496 and 498/500 extend upwardly from the raised segment 486 behind the aperture 416 and from the raised segment 488 behind the aperture 418.

Figures 66 to 69 show a cassette guide 510 for use in guiding the installation of the assembled cassette 302 into the proper location for latching on the pump chassis 370. At the rear the cassette guide 510 has an aperture 512 on the right and an aperture 514 on the left. The aperture 512 will be aligned with one of the threaded apertures 404, 408 or 412 (Figure 62) while the aperture 514 will be aligned with one of the threaded apertures 402, 406 or 410, to install the cassette guide 510 in either the first, second, or third position.

The top side (Figure 66) of the cassette guide 510 has a rectangular recess 516 which corresponds in size to the rectangular recesses 420, 422 and 424 in the pump chassis 370. The sensor modules will be accommodated between the rectangular recesses 516 in the cassette guides 510 and the rectangular recesses 420, 422, and 424 in the pump chassis 370. The right hand side of this rectangular recess 516 is exposed through a rectangular aperture 518 in the cassette guide 510 (Figure 67).

An area 520 on the bottom of the cassette guide 510 immediately in front of the rectangular aperture 518 and an area 522 immediately to the right and to the rear of the rectangular aperture 518 is recessed upwards from the under surface 524 of the cassette guide 510. At the front right corner of the rectangular aperture 518, a square segment 528 extends downwards to the level of the bottom surface 524 of the cassette guide 510. Located immediately forward of the square segment 528 is a thin rectangular track 530 extending from the right side of the cassette guide 510. The thin rectangular track 530 terminates at its front end in a blocking segment 532.

The front end of the cassette guide 510 has a rounded notch 534, which is positioned to receive the outlet tube mounting cylinder 144 on the cassette body 100 (Figure 4) when the cassette guide 510 is installed on the pump chassis 370. When the cassette guide 510 in installed on the pump chassis 370, the rear-most portion of the assembled cassette 302 will fit between the cassette guide 510 and the bottom of the pump chassis 370. Accordingly, the cassette guide 510, together with the various support walls on the bottom of the pump chassis 370, aids in the installation of the assembled cassettes 302 in the proper position for latching.

Figure 70 shows a pump shaft 540 which is essentially cylindrical. Near the top end of the pump shaft 540, at the front, a cam follower wheel 542 is mounted for rotation about a short axle 544 extending orthogonally from the pump shaft 540. On the rear side of the pump shaft 540 at the same location, an alignment wheel 546 is mounted for rotation about a short axle 548 extending orthogonally from the pump shaft 540 opposite the short axle 544. Near the bottom end of the rear of the pump shaft 540 there is conical recess 550, which will be used to attach the jaws assembly 360 (Figure 59 to 61) to the pump shaft 540.

Figures 71 to 76 show a slide lock 560 which is for mounting on the thin rectangular track 530 of the cassette guide 510 (Figure 67). the slide lock 560 has a U-shaped slide channel 562 at the front, with the open portion of the U facing left and extending from front to rear. On the other side of the slide channel 652 (which is the bottom of the U), there is a rectangular notch 564 located near the front which runs from the top to the bottom of the slide channel 562 and faces right.

Extending back from the rear of the slide channel 562 at the bottom, there is a thin rectangular connecting segment 566, which effectively extends from the leg of the U at the bottom of the slide channel 562. Attached at the rear edge of the segment 566 there is a U-shaped channel 568 with the open portion of the U facing right and extending from top to bottom. The top of the forward leg of the U of the U-shaped channel 568 is attached to the segment 566. It will be appreciated that the top surface of the rectangular connecting segment 566 and the top of the U-shaped channel 568 are coplanar, and that the interior surface of the lowermost leg of the slide channel 562 is also coplanar.

The upper left edge of the U-shaped channel 568 is bevelled at 570. The function of the bevel 570 is to serve as a light reflector, as will be come apparent later in conjunction with the discussion of the mechanism for latching the assembled cassette 302.

Figures 77 to 79 show an essentially cylindrical power module cam 580. The power module cam 580 has a central bore 582 therethrough for mounting the cam 580 on a shaft (not shown). The cam 580 has apertures 584 and 586 through which means for retaining it in position on a shaft may be installed. Located near to the bottom of the cam 580, there is a groove 588 located around its outer circumference. The groove 588 will receive a drive belt which will drive the cam 580 in a rotary fashion.

Located above and spaced slightly away from the groove 588, there is a retaining groove indicated generally at 590 formed in the surface of and extending around the circumference of the power module cam 580. The retaining groove 590 is of essentially uniform width and depth in the surface of the cam 580, but varies in its distance from the top. As best seen in Figure 77, the portion of the retaining groove 590 closest to the top of the cam 580 is disposed approximately one-hundred-eighty degrees away from the portion of the retaining groove 590 furthest from the top of the cam 580. It will be understood that a non-rotating member having a portion engaged in the retaining groove 590 will be driven in a reciprocating fashion as the power module cam 580 is rotated.

A cam surface indicated generally at 592 is formed in the base of the power module cam 580 about its outer diameter. The cam surface 592 extends lower in one portion 593 than in the other portion 595, as best shown again in Figure 77. It will be apparent to those skilled in the art that one or more non-rotating members bearing on the cam surface 592 will be driven in a reciprocating fashion as the power module cam 580 is rotated.

The configurations of the retaining groove 590 and the cam surface 592 are graphically illustrated in Figure 80, which indicates how three members driven by the power module cam 580 are caused to operate as the power module cam 580 is rotated through a three-hundred-sixty degree cycle. The retaining groove 590 is used to drive a pump member, which draws fluid in from a source to fill the pump chamber on an intake stroke, and pumps the fluid out on a pumping stroke. The cam surface 592 is used to drive two valve members, namely an inlet valve and an outlet valve which are driven by portions of the cam surface 592 which are separated by approximately one-hundred-eighty degrees. It will at once be appreciated that the pump and valves being driven will be those of the assembled cassette 302.

The plot of pump displacement in Figure 80 illustrates that there is a fill cycle during which displacement increases from zero (or near zero) to full, and a pump cycle during which displacement decreases from full to empty (or near empty). The retaining groove 590 has two flat portions which correspond to the flat portions of the pump displacement plot. One of the flat portions 594 is the portion of the retaining groove 590 which is closest to the top, and this flat portion 594 corresponds to the zero displacement portion of the pump displacement plot.

The other flat portion 596 is the portion of the retaining groove 590 which is closest to the bottom and this flat portion 596 corresponds to the full displacement portion of the pump displacement plot.

The portions of the retaining groove 590 which are located intermediate the flat portion 594 and 596 are a positive portion 598 which corresponds to the increasing displacement portion of the pump displacement plot, and a negative portion 600 which corresponds to the decreasing displacement portion of the pump displacement plot. It should be noted that the flat portions 594 and 596 are substantial enough to allow complete valve movement during the flat portions of the pump displacement plot. In the preferred embodiment, the flat portions 594 and 596 each represent approximately sixty degrees of rotational movement, while the positive and negative portions 598 and 600 each represent approximately one-hundred-twenty degrees of rotational movement.

The cam surface 592 of the power module cam 580 is described with reference to the inlet and outlet valve plots of Figure 80. It will first be noted that the plots for the inlet and outlet valves are identical, but are located one-hundred-eighty degrees apart. As will become evident later in conjunction with the discussion of the valve actuators and the valve actuator guide, the inlet and outlet valves are both driven by the cam surface 592, but by points on the cam surface which are located one-hundred-eighty degrees apart.

The lower portion 593 of the cam surface 592 corresponds to the closed positions of both the inlet and outlet valves, while the higher portion 595 of the cam surface 592 corresponds to the opened positions of the valves. All valve movement is accomplished entirely during the periods in which pump displacement remains constant. In the preferred embodiment, where pump displacement is constant during two sixty degree periods and either increasing or decreasing during two one-hundred-twenty degree periods, all valve movement is accomplished during the two sixty degree periods.

In addition, at least one valve is closed as any given time to prevent free flow through the assembled cassette 302. Therefore, it will be appreciated that the period during which the inlet and outlet valves have their transition between the fully open and closed positions will be limited to thirty degrees or less in the preferred embodiment. During each of the sixty degree periods during which pump displacement is constant, the one of the valves which is open will close, and only then will the other valve, which was closed, be allowed to open.

Figure 81 shows a drive module assembly 602 including the power module cam 580 discussed above. The various parts described in Figure 81 are mounted onto a drive module chassis 604, which will in turn be mounted onto one of the three pump positions on top of the pump chassis 370. As shown in Figure 82, the drive module chassis 604 has an aperture 605 on the left had side and two apertures 607 and 609 on the right hand side. The apertures 605, 607 and 609 are for use in fastening the drive module assembly 602 to the pump chassis 370.

An ironless core DC motor 606 is used to drive the system. The motor 606 typically has a built-in gear reduction unit to reduce its output speed. The motor 606 is mounted on the top surface of the drive module chassis 604 at one side, the output shaft extends through the drive module chassis 604. A drive pulley 608 is mounted on the output shaft and is thus driven by the motor 606.

A one-way clutch 610 is mounted onto the top of the drive module chassis 604 at the other side. Such devices are commercially available, and are known as DC roller clutches or overrunning clutches. The one-way clutch 610 supports a drive shaft 612 for rotation, with both ends of the drive shaft 612 extending from the one-way clutch 610. The one-way clutch 610 allows the drive shaft 612 to rotate in one direction only; in the preferred embodiment, the rotation is clockwise when viewed from the top. The power module cam 580 is mounted on the bottom end of the drive shaft 612 extending from the one-way clutch 610. A drive belt 613 is mounted over the drive pulley 608 and in the groove 588 in the power module cam 580. The motor 606 will thereby drive the power module cam 580 and the drive shaft 612.

An angular incremental position sensor 614 is fixedly mounted above the one-way clutch 610. A sensor disc 616 is fixedly mounted on the top end of the drive shaft 612, and rotates with the drive shaft 612 and the power module cam 580. The position sensor 614 is used to provide angular incremental and absolute position feedback for control of the drive mechanism and cassette. In the preferred embodiment, the position sensor 614 should also be capable of direction sensing.

Figures 85 to 87 show a valve actuator 620 which is driven by the power module cam 580. The valve actuator 620 includes a thin, essentially rectangular portion 622 with a circular bearing 624 rotatably mounted near the top. The circular outer diameter of the bearing 624 extends slightly about the top of the rectangular portion 622. The bearing 624 is the portion of the valve actuator 620 which will be in contact with the cam surface 592 of the power module cam 580.

The rectangular portion 622 of the valve actuator 620 is chamfered on its edges as indicated generally at 625, and has a small notch 626, 628 in both its lateral sides at a location above the lower end. The small notches 626 and 628 are for receiving means for retaining the valve actuator 620 in position once it is installed; this will become evident below in conjunction with the discussion of the assembly of the main pump unit.

Figures 83 and 84 show a valve actuator guide 630 which is used to guide and retain in position pairs of the valve actuators 620. The upper portion 632 of the valve actuator guide 630 is square in cross-section, and the lower portion 634 is circular in cross-section. Extending vertically through both the upper and lower portions 632, 634 are two rectangular apertures 636 and 638, which are each sized to allow the rectangular portion 622 of a valve actuator 672 to slide freely therein.

One valve actuator guide 630 will be installed at each of the pump positions in the pump chassis 370. In the first pump position, the square upper portion 632 of the valve actuator guide 630 will be located in the square aperture 438 on the pump chassis 370 and the circular lower portion 634 of the valve actuator guide 630 will be located in the circular recess 432 on the pump chassis 370. In the second pump position, the square upper portion 632 will be located in the square aperture 440 and the circular lower portion 634 will be located in the circular recess 434. In the third pump positions the square upper portion 632 will be located in the square aperture 442 and the circular lower portion 634 will be located in the circular recess 436.

Figures 88 to 90 show a pressure transducer 660, one of which will be installed in the pump chassis 370 in each pump position, in the circular recesses 444, 446, and 448. The pressure transducer 660 is essentially cylindrical, with a circumferential groove 662. The groove 662 receives an elastomeric O-ring (not shown) which will both retain the pressure transducers 660 in the circular recesses 444, 446, and 448 and provide a fluid seal. Located on top of the pressure transducer 660 is a square segment 664 in which is located the actual transducer, which square segment 664 will be received in the cylindrical apertures 450, 452, and 454. Extending upwards from the square segment 664 are several leads 666.

Figures 91 to 96 show an optical sensor module 670. The optical sensor module 670 is essentially rectangular in cross-section, with a wider rectangular flange 672 on top of the rectangular portion and a generally oval portion 674 above the rectangular flange 672. A flex or cable 676 extends from the top of the oval portion 674. Located around the circumference of the oval portion 674 is a groove 678, which will retain the oval portion 674 of the optical sensor modules 670 in the oval apertures 426, 428 or 430. The rectangular flange 672 of the optical sensor modules 670 will fit into the rectangular recesses 420, 422 or 424 in the first, second, or third pump positions, respectively.

The rectangular portion of the optical sensor module 670 has a notch 680 in the front immediately under the rectangular flange 672, which will receive the rear most portion of the assembled cassette 302. The underside of the rectangular portion of the optical sensor module 670 has an optical light source 682 and an optical light sensor 684 at locations near and equidistant from the right hand side. The optical light source 682 and the optical light sensor 684 are used to detect when the slide lock 560 is in the closed position, as will be discussed below.

Located in the downwardly-facing upper surface of the notch 680 in the optical sensor module 670 are three optical light sources 686, 688 and 690 which extend in a line from left to right. Immediately below the three optical light sources 686 , 688 and 690, on the upwardly-facing lower surface of the notch 680 near the right hand side there are three optical light sensors 692, 694, and 696 which also extend in a line from left to right. The three optical light sources 686, 688, and 690 and the three optical light sensors 692, 694, and 696 are used to provide the three cassette indentification bits as will be discussed below.

Also located on the lower surface of the notch 680 towards the left hand side, there is an optical light source 698 and in front of the optical light source 698, an optical light sensor 700. The optical light source 698 and the optical light sensor 700 are used to detect the presence of (or absence) of an air bubble in the fluid line in the cassette. The location of the optical light source 698 and the optical light sensor 700 as illustrated in Figure 967 is that of the preferred embodiment, and the operation of that preferred embodiment as well as the configurations and operational description of several alternative embodiments are discussed below.

Figures 97 and 98 show a valve actuator seal 650 which provides a fluid sal and, more importantly, retains the valve actuators 620 (Figures 85 to 87) in an upright position with their bearings 624 against the lower portion 593 of the power module cam 580. The outer circumference of the valve actuator seals 650 is of a size allowing them to be retained with a friction fit in the circular recesses 432, 434, and 436 below the valve actuator guides 630. A metal ring (not shown) may be moulded into the outer diameter of the valve actuator seals 650 to improve their retention in the circular recesses 432, 434, and 436.

Two apertures 652 and 654 which are rectangular in configuration, are located in the valve actuator seal 650 to receive the ends of the rectangular portion 622 of the valve actuators 620. The lengths of the apertures 652 and 654 are shorter than the width of the rectangular portion 622 of the valve actuators 620, with the small notches 626 and 628 in the rectangular portion 622 in each case being used to capture the end of one of the apertures 652 and 654. It will be appreciated that the small notches 626 and 628 of the valve actuators 620 will engage the apertures 652 and 652 in the valve actuator seal 650, thereby allowing the valve actuator seal 650 to exert a bias on the valve actuators 620. As will be seen below, the bias exerted by the valve actuator seal 650 on the valve actuators 620 is an upward one, urging the valve actuators 620 against the lower portion 583 of the power module cam 580.

In the previous discussions of the various parts of the main pump unit, the function and interrelationship between parts has been briefly discussed. Before. moving on to the operation of the main pump unit and the assembled cassette 302 a brief discussion of the assembly of the main pump unit is in order. This discussion specifically refers to Figures 62 to 65 ( the pump chassis 370), Figure 99 and Figure 112 and also to other figures which are specifically mentioned in the discussion.

A pump shaft bearing 640 is installed in both the top and the bottom of each of the apertures 414, 416, 418 in the pump chassis 370. The pump shaft bearings 640 (Figure 112) are essentially cylindrical and have a cylindrical bore. In the preferred embodiment bearings 640 have a collar 641 at one end and fit in the apertures 414, 416 and 418 from the top and from the bottom with an interference fit to retain them in position. The pump shaft bearings 640 are preferably made of a low friction material such as Teflon to allow the pump shafts 540 to move freely. It will also be appreciated that a single bearing could be used in each of the apertures 414, 416, 418, extending all the way through the apertures 414, 416, 418.

Next, the valve actuator guides 630 (Figures 83 and 84) are installed from the bottom of the pump chassis 370 into the circular recess 432 and the square aperture 438 in the first pump position, into the circular recess 434 and the square aperture 440 in the second pump position, and into the circular recess 436 and the square aperture 442 in the third pump position. With the valve actuator guides 630 installed, the bottom surface of each valve actuator guide 630 leaves a portion of the corresponding circular recess 432, 434, and 436 open from the bottom side of the pump chassis 370. The valve actuator seals 30 (Figures 97 and 98) will be installed later in the circular recesses 432, 434, and 436 below the valve actuator guides 630.

The next step in this assembly is to install the two sensor modules. The pressure transducers 660 (Figures 88 to 90) are installed from the bottom of the pump chassis 370 into the circular recesses 444, 446, 448. The pressure transducers 660 are essentially cylindrical and with O-rings in the grooves 662 fit snugly into the circular recesses 444, 446, 448 with their bottoms surfaces flush with the bottom surface of the pump chassis 370 around the circular recesses 44, 446 and 448. The upper surface of the cylindrical portion of the pressure transducers 660 fit against the cylindrical apertures 450, 452, and 454 in the pump chassis 370. Although not shown in the drawings, a surrounding portion of the bottom surface of the pump chassis 370. This thin membrane protects the pressure transducer 660 from fluids which may inadvertently or accidentally come in to contact with the device.

Optical sensor assembles 670 (Figures 91 to 96) are installed in the rectangular recesses 420, 422, and 416 of the pump chassis 370 fitting into the oval apertures 426, 428 and 430. the optical sensor modules 670 are retained in position by the pressure of O-rings in the grooves 678 in the optical sensor modules 670, and by the cassette guides 510.

The next step in the the assembly of the main pump unit mechanical components on the pump chassis 370 is the installation of the cassette guide 510 (Figures 66 to 69) and the slide lock 560 (Figures 72 to 76). The slide lock 560 is installed ont o the cassette guide 510 by placing the portion of the slide lock 560 including the bottom of the slide channel 562 into the rectangular aperture 518 in the cassette guide 510 from above, with the rectangular connecting segment 566 of the slide lock 560 extending over the portion of the area 522 at the rear of the cassette guide 510. This aligns the interior of the U-shaped slide channel 562 on the slide lock 560 with the back end of the thin rectangular track 530 on the cassette guide 510. The slide lock 560 is then moved forwards with respect to the cassette guide 510, so that the interior of the slide channel 562 fits over the thin rectangular track 530 until the blocking segment of the cassette guide 510 is contacted by the slide lock 560.

The cassette guides 510 together with the slide locks 560 may then be mounted in the three pump positions on the pump chassis 370, which already contain the optical sensor module 670, using two screws (not shown). In the first pump position, a screw is inserted through the aperture 514 in the cassette guide 510 into the threaded aperture 402 in the pump chassis 370, and a second screw is inserted through the aperture 512 in the cassette guide 510 into the threaded aperture 404 in the pump chassis 370. In the second position, screws are inserted through the apertures 514 and 512 into the threaded apertures 406 and 408 respectively, and in the third pump position, screws are inserted through the apertures 514 and 512 into the threaded apertures 410 and 412 respectively. By way of example, the cassette guide 510 and the slide lock 560 are shown mounted in the first pump position in Figure 99.

Next, the pump shafts 540 are installed in the pump shaft bearings 640, which have previously been installed in the apertures 414, 416 and 418. The end of the pump shafts 540 containing the conical recess 550 are inserted through the pump shaft bearings 640 from above, with the alignment wheel 546 being located between one of the three pairs of guide fingers, namely the guide fingers 490 and 492 for the first pump position, the guide fingers 494 and 496 for the second pump position, and the guide fingers 498 and 500 for the third pump position. The pump shaft 540 is shown installed in the first pump position in Figure 112.

The valve actuators 620 are installed next, with one pair of the valve actuators 620 being installed in each pump position. The bottom ends of the valve actuators 620 having the chamfered edges 625 are inserted through the top sides of the valve actuator guides 630, with one pair of valve actuators 620 being installed in each of the three valve actuator guides 630. The pair of valve actuators 620 are inserted into the apertures 636 and 638 in the valve actuator guides 630 with the bearings 624 on each actuator 620 facing away from each other.

It will be appreciated that the rectangular portions 622 of the valve actuators 620 will extend downwards through the apertures 636 and 638 in the valve actuator guides 630. As stated above, a valve actuator seal 650 is used in each of the three pump positions, mounted from beneath the pump chassis 370 into the circular recesses 432, 434 and 436 below the valve actuator guides 630. The outer circumference of the valve actuator seals 650 causes them to be retained with a friction fit in the circular recess 432, 434, and 436.

The lower ends of the rectangular portions 622 of each pair of the valve actuators 620 extend downwards through the apertures 652 and 654 in the respective valve actuator seal 650. The small notches 626 and 628 in one valve actuator 620 is retained in the aperture 652 in the valve actuator seal 650, and the other valve actuator 620 is similarly retained in the aperture 654. As shown in Figures 113 and 114 the valve actuator seals 650 will tend to urge the valve actuators 620 in an upward direction. In the preferred embodiment the bottoms of the valve actuators 620 having the chamfered edges 625 will protrude somewhat from the bottom surface of the pump chassis 370 around the circular recesses 432, 434 and 436 even when the valve actuators 620 are in their open position. For example in their open position they may protrude approximately thirty thousands of an inch (0.8mm), and in their closed position they may protrude seventy thousands of an inch (l.8mm).

This upward biasing of the valve actuator 620 is essential both to allow the assembled cassettes 302 to be freely inserted, and to maintain the valve actuators 620 in an upward position with their bearings 624 against the lower portion 593 of the power module cam 580. The valve actuator seals 650 accordingly function both to provide a fluid seal and to bias the valve actuators 620 to the upward position described.

The next step in the assembly of the main pump unit is to install a drive module assembly 602 (Figure 81) onto each of the three pump positions on the pump chassis 370. In the first pump position, the drive module assembly 602 will be supported above the top of the pump chassis 370 by the cylindrical raised segment 456 and the oval raised segment 458. Three screws (not shown) will be used to secure the drive module assembly 602 in the first pump position, the first screw being inserted through the aperture 605 in the drive module chassis 604 into the threaded aperture 466 in the pump chassis 370, the second being inserted through the aperture 607 in the drive module chassis 604 into the threaded aperture 468 in the pump chassis 370, and a third being inserted through the aperture 609 in the drive module chassis 604 into the threaded aperture 470 in the pump chassis 370. In the first pump position, the power module cam 580 is supported directly above the square aperture 438 in the pump chassis 370, the valve actuator guide 630 and the two valve actuators 620 located in the first pump position.

In the second pump position, the drive module assembly 602 will be supported above the top of the pump chassis 370 by the oval raised segment 458 and the oval raised segment 460. Three screws (not shown) will be used to secure the drive module assembly 602 in the second pump position, with the first screw being inserted through the aperture 605 into the threaded aperture 472 in the pump chassis 370, the second being inserted through the aperture 607 into the threaded aperture 474 and the third being inserted through the aperture 609 into the threaded aperture 476. In the second pump position, the power module cam 580 is supported directly above the square aperture 440 in the pump chassis 370, the valve actuator guide 630 and the two valve actuators 620 located in the second pump position.

In the third pump position, the drive module assembly 602 will be supported above the top of the pump chassis 370 by the oval raised segment 460, the cylindrical raised segment 462, and the cylindrical raised segment 464. Three screws (not shown) will again be used to secure the drive module assembly 602 in the third pump position, the first screw being inserted through the aperture 605 into the threaded aperture 478, the second being inserted through the aperture 607 into the threaded aperture 480 and the third being inserted through the aperture 609 in the drive module chassis 604 into the threaded apeture 482. In the third pump position, the power module cam 580 is supported directly above the square aperture 442 in the pump chassis 370, the valve actuator guide 630 and the two valve actuators 620 located in the third pump position.

The final component to be installed is the jaws assembly 360 (Figures 59 to 61), with one jaw assembly 360 being installed in each of the three pump positions and engaging the bottom of the pump shafts 540, which are located in the apertures 414, 416 and 418. The bottom end of the pump shaft 540 with its conical recess 550 is inserted into the cylindrical aperture 316 in the latch head 310 of the jaws assembly 360. A retaining screw (not shown) is screwed into the threaded aperture 318 in the latch head 310, and into the conical recess 550 of the pump shaft 540 to retain the jaws assembly 360 in place on the bottom of the pump shaft 540.

The location of the installed jaws assembly 360 is shown in Figure 99, with the slide lock 560 and the latch jaw 340 in the open position. The link pin 354 on the latch jaw 340 is located in the U-shaped channel 568 of the slide lock 560, and movement of the slide lock 560 will accordingly cause the latch jaw 340 to move. When the slide lock 560 is fully forward, as shown in Figure 99, the latch jaw 340 will be in the open position, with the jaw portion 342 of the latch jaw 340 away from the right jaw 314 of the latch head 310. When the slide lock 560 is pushed towards the back of the pump chassis 370, as shown in Figure 100, the latch jaw 340 will be in the closed position, with the jaw portion 342 of the latch jaw 340 closely adjacent the right jaw 314 of the latch head 310.

This completes the discussion of the assembly of the main pump unit with three pump positions. It is, of course, within the scope of the present invention to employ a main pump unit with different numbers of pump positions.

The installation of the assembled cassette 302 at the first pump position, and the operation of the device to pump fluid and to perform the other associated functions will now be described. The operation of the other two pump positions are identical to the operation of the first pump position described below.

With the slide latch 240 pulled back fully away from the front of the assembled cassette 302 (Figure 43 to 48), the wider portion of the elongate, tear-shaped aperture 258 in the slide latch 240 will close the tube 306, preventing fluid from flowing though the assembled cassette 302. The inlet tube 304 is connected to a fluid source such as an IV bag (not shown), and the outlet tube 306 is connected to a fluid delivery device such as an injection set (not shown), the use of which is well known in the art. The slide latch 240 is opened, together with any other closures in the IV bag line, and fluid fills the lines, the assembled cassette 302, and the injection set. By tapping or shaking the assembled cassette 302 any residual air bubbles will flow out through the line. The slide latch 240 is then pulled back and the outlet tube 306 is closed, and the system is in a primed condition with the assembled cassette 302 ready to be installed onto the main pump unit.

When the slide latch 240 is pulled back, an opening is left between the front portion 242 of the slide latch 240 and the front top portion of the assembled cassette 302 (made up of the cassette body 100 and the retainer cap 190) facing the front portion 242 of the slide latch 240. In this example, where the assembled cassette 302 is to be mounted in the first position, the opening between the font portion 242 of the slide latch 240 and the front top portion of the assembled cassette 302 will admit the fist pair of angled segments 372 and 374 as the assembled cassette 302 is installed. The top surface of the assembled cassette 302, which is the retainer cap 190 (Figure 43), will abut against the bottom of the pump chassis 370 (Figure 62)

Prior to installing the assembled cassette 302 into the main pump unit, the slide lock 560 must be fully forward with the latch jaw 340 opened away from the latch head 310, as mentioned previously and as shown in Figure 99. In addition, the jaws assembly 360 should be in its fully upward position, which may be achieved by using the motor 606 to drive the power module cam 580 to cause the jaws assembly 360 to be driven to this position using the position sensor 614.

The rear-most edge of the assembled cassette 302 is tilted upwards then placed against the bottom of the pump chassis 370 between the pressure transducer 660 (mounted flush with the bottom of the pump chassis 370) and the top side of the cassette guide 510. The rear-most portion of the top of the assembled cassette 302 is slid towards the back of the pump chassis 370 into position between the left lateral support wall 384 on the left and the right lateral support walls 390 on the right, with most of the rear-most portion of the top of the assembled cassette 302 fitting into the notch 680 in the optical sensor module 670. The upper right back corner of the assembled cassette 302 is supported positioned by the right corner support wall 396, which contacts the rear of the assembled cassette 302 behind the pump cylinder 112 and the portion of the right side of the assembled cassette 302 adjacent the pump cylinder 112.

When the assembled cassette 302 is pushed fully back into place, the front of the assembled cassette 302 is tilted upward against the bottom of the pump chassis 370, with the first pair of angled segments 372 and 374 on the bottom of the pump chassis 370 fitting into the area between the front portion 242 of the slide latch 240 and the front top portion of the assembled cassette 302. The slide latch 240 is then pushed into the cassette body 100, sliding the inverted L-shaped portion 250 of the slide latch 240 into engagement with the angled segment 372, and sliding the inverted, backwards L-shaped portion 252 of the slide latch 240 into engagement with the angled segment 374. The assembled cassette 302 will thus be held in position on the bottom of he pump chassis 370 until the slide latch 240 is again pulled back, releasing the assembled cassette 302.

Simultaneously, the outlet tube 306 will be opened, but fluid will not flow through the outlet tube 306 since at least one of the valve actuators 620 will be in its fully downward position at any given time, thereby preventing free flow through the assembled cassette 302 whenever the assembled cassette 302 is installed on the main pump unit. It will also be noted that in this initially installed position, the piston cap portion 262 is located at the very top of the pump cylinder 112.

It will be appreciated as discussed above that the power module cam 580 will operate both the reciprocations of the piston assembly 280 and the movement of the valve actuators 620A and 620B (Figure 112). The movement of the piston assembly 280 and the valve actuators 620A and 620B will correspond to the charts of Figure 80, with the initially installed position corresponding roughly to the zero degree position in the charts. In this position, both the inlet valve actuator 620A and the outlet valve actuator 620B are in their closed positions.

Note that the open positions of the inlet valve actuator 620A and the outlet valve actuator 620B are their fully upward positions, and that their closed positions are their fully downward positions. If both the inlet valve actuator 620A and the outlet valve actuator 620B were simultaneously not in place on the domed portion 178 of the valve diaphram 170 of the assembled cassette 302, then the area including the first passageway 128, the smaller diameter aperture 118 to the pump cylinder 112, and the second passageway 134 would be entirely open and fluid flow would be unrestricted.

When the inlet valve actuator 620A is in its closed or fully downward position, the portion of the domed portion 178 located between the first passageway 128 and the smaller diameter aperture 118 is forced down on to the portion of the slightly raised border 146 between the first passageway 128 and the smaller diameter diameter aperture 118, thereby preventing fluid flow between the first passageway 128 and the smaller diameter aperture 118. This position of the inlet valve actuator 620A is referred to as its closed position.

Similarly, when the outlet valve actuator 620B is in its closed or fully downward position, the portion of the domed portion 178 located between the smaller diameter aperture 118 and the second passageways 134 is forced down onto the portion of the slightly raised border 146 between the smaller diameter aperture 118 and the second passageway 134, thereby preventing fluid flow between the smaller diameter aperture 118 and the second passageway 134. This position of the outlet valve actuator 620B is referred to as its open position.

The motor 606 will begin to drive the power module cam 580, causing the inlet valve actuator 620A to open, with the outlet valve actuator 620B remaining closed, as shown in Figure 113. As the power module cam 580 continues to be turned by the motor 606 , the piston cap portion 262 will be drawn downwards in the pump cylinder 112, causing fluid to be drawn into the pump cylinder 112 from the fluid source (not shown) through the inlet tube 304, the bubble trap 104, and the first passageway 128. When the pump cylinder 112 is filled, the inlet valve actuator 620A is closed. Only after the inlet valve actuator 620A is fully closed will the outlet valve actuator 620B be opened. Figure 114 shows the system with the outlet valve actuator 620B opened, prior to any fluid being pumped out. The main pump unit responds to an electronic control system (not shown) which operates the system. This electronic control system, which is preferably microprocessor-based, may be either conventional as known in the art, or it may differ to enhance the unique mechanical design of the system discussed herein.

Fluid will be pumped by the motor 606 turning the power module cam 580, to drive the piston cap portion 262 upwards in the cylinder, forcing fluid out of the cylinder 112, and out of the assembled cassette 302 through the outlet tube 306, from which it is supplied to the patient through the injection set (not shown). It will be appreciated by those skilled in the art that the system may pump fluid at any rate chosen, by operating the motor 606 at any given rate. In addition, the use of the position sensor 614 will provide a feedback signal indicating the exact position of the power module cam 580 and the piston assembly 280, thereby indicating precisely how much fluid has been pumped by the device.

As noted previously, the rear-most portion of the assembled cassette 302 is located in the notch 680 of the optical sensor module 670 when the cassette is installed in the main pump unit. This is illustrated in Figures 101 and 102, which show only the assembled cassette 302 and the optical sensor module 670. In some situations it may be desirable to use several different types of assembled cassettes 302 with the system described herein. For example, different cassettes may require different stroke volumes to fittings on the inlet tube 304 and/or the outlet tube 306 of the cassettes. Special application cassettes such as internal pump cassettes, continuous arteriovenous hemofiltration (CAVH) cassettes, continuous blood sampling cassettes, or autotransfusion cassettes may be manufactured.

The use of the wrong cassette may present a high degree of danger, so it will be perceived that it is highly desirable to identify the particular cassette installed. This may be accomplished by the use of the three cassette identifying indicia 148, 150 and 152. By making each of these indicia a binary bit, up to eight different codes may be generated. By using redundant coding to ensure fail-safe operation, three different cassettes can be identified. In addition, the absence of a cassette can also be detected. In the example illustrated in the drawings, the first and third cassette identifying indicia 148 and 152 are of a first type (identified as logic ONE for convenience), and the second cassette identifying indicia 150 is of a second type (identified as logic ZERO for convenience).

With the assembled cassette 302 installed with its rear-most portion located in the notch 680 of the optical sensor module 670, the first indicia 148 is aligned with the first pair of sensor elements, namely the optical light source 686 and the optical light sensor 692. Similarly, the second indicia 150 is aligned with the second pair of sensor elements, namely the optical light source 688 and the optical light sensor 694. Likewise, the third indicia 152 is aligned with the third pair of sensor elements, namely the optical light source 690 and the optical light sensor 696.

The second cassette identifying indicia 10 (logic ZERO) and the second pair of sensor elements are shown in Figure 103. Light from the optical light source 688 shines through the aperture 208 in the retainer cap 190, and onto the cassette body 100, where it is dispersed by the second cassette identifying indicia 150, which comprises an inverted V moulded into the bottom of the upper surface 102 of the cassette body 100. Various prism types of construction could also be used to disperse the light, so that it does not reach the optical light sensor 694, resulting in a logic ZERO being output by the optical light sensor 694. The inverted V could be moulded into the top side of the upper surface 102 of the cassette body 100, while other alternatives include using paint or some other physical blocking expedient instead of a dispersing lens, or selectively moulding or not moulding one or more of the apertures 206, 208 and 210 in the retainer cap 190 (Figures 13 and 14).

The third cassette identifying indicia 152 (logic ONE like the first cassette identifying indicia 148, which is not shown here) and the third pair of sensor elements are shown in Figure 104. Light from the optical light source 690 shines through the aperture 210 in the retainer cap 190, and onto the third cassette identifying indicia 152 on the cassette body 100. The third cassette identifying indicia 152 is a cylindrical projection which extends up from the upper surface 102 of the cassette body 100, and which acts as a light guide to conduct the light to the optical light sensor 696, where it causes the optical light sensor 696 to generate a logic ONE output. In the preferred embodiment, the cassette body 100 is constructed of a clear plastics material to allow the first cassette identifying indicia 148 and the third cassette identifying indicia 152 to conduct the light. Also in the preferred embodiment, when there is no cassette 302 in place, all three outputs are logic ONES and this signal is used to indicate that no cassette has been installed or that the cassette 302 is improperly installed.

It will therefore be appreciated that the use of the three cassette identifying indicia 148, 150 and 152 allows the generation of three digital cassette identifying signals which are supplied from the optical sensor module 670 to the microprocessor (not shown) to identify the particular type of cassette which is installed. By using this cassette identifying system, inappropriate use of an installed cassette and/or improper cassette installation may be prevented.

It is also desirable to provide an indication that the assembled cassette 302 has been properly installed on the main pump unit, with the latching mechanism properly closed. This occurs when the slide lock 560 is pushed fully back against the rear of the cassette guide 510. This is accomplished by sliding the slide latch 240 fully into the assembled cassette 302, with the tab 257 on the slide latch 240 fitting into the notch 564 on the slide lock 560 to drive the slide lock 560 back, thereby also latching the jaws assembly 360 onto the piston assembly 280.

An indication of latching is provided through use of the optical light source 682 and the optical light sensor 684 on the bottom of the optical sensor module 670. When the slide lock 560 is in its loading or forward position shown in Figure 99, the bevel 570 on the slide lock 560 is adjacent the optical light source 682 and the optical light sensor 684 on the bottom of the optical sensor module 670, as shown in Figures 105 and 106. The presence of the bevel 570 reflects the light coming from the optical light source 682 to the right, away from the optical light sensor 684, thereby preventing a latch closed signal. When the slide lock 560 is pushed fully back to its closed or rear-most position shown in Figure 100, the bevel 570 on the slide lock 560 is not adjacent the optical light source 682 and the optical light sensor 684, as seen in Figure 107. Rather, a reflective surface 567 installed on the flat bottom of the rectangular connecting segment 566 of the slide lock 560 reflects light from the optical light source 682 into the optical light sensor 684, thereby generating a latch closed signal. The reflective surface 567 acts as a mirror, and may be a foil segment which could be hot stamped into the rectangular connecting segment 566 or adhesively secured to the bottom of the rectangular connecting segment 566.

Additional confirmation that the slide lock 560 is closed with an assembled cassette in place may be obtained by verifying the cassette identifying indicia, as described above. In order to give an absolutely positive confirmation that a cassette is properly installed and that the slide lock 560 is in the closed position, the preferred embodiment will required correct signals from both the optical light sensor 684 and from the optical light sensors 692, 694 and 696.

One of the essential functions of the system is to enable the detection of air in the fluid line of the system. The air in line detection (AILD) system of the preferred embodiment is shown in Figure 108, and includes the recessed lens portion 138 in the assembled cassette 302, and a pair of sensor elements, namely the optical light sorce 698 and the optical light sensor 700, in the optical sensor module 670. The recessed lens portion 138 is an optical viewing area in the fluid pathway through the assembled cassette 302, and in the preferred embodiment shown in Figure 108 is an inverted prism. The recessed lens portion 138 in this and other embodiments also includes a focusing lens, indicated generally at 697. The optical light source 698 and the optical light sensor 700 are both mounted in the optical sensor module 670 below the recessed prismatic lens portion 138 in the installed cassette 302.

The optics of the system of Figure 108 makes use of the properties of light as it moves from one medium to a less dense medium, and is a "reverse reflected" configuration. When there is air in the fluid channel, the light from the optical light source 698 follows the path shown in Figure 108, undergoing total internal reflection at the surfaces of the recessed prismatic lens portion 138 so that it is directed downwards to the the optical light sensor 700. Even if the upper surfaces of the recessed prismatic lens portion 138 are wetted with a liquid film, total internal reflection still occurs. When there is liquid in the channel, the light refracts through the recessed prismatic lens portion 138 into the liquid. If the liquid is clear, the light passes through the liquid, where it is either absorbed by the valve diaphragm 170 or the retainer cap 190, or passes through both the valve diaphragm 170 and the retainer cap 190. Accordingly, the valve diaphragm 170 may be clear, absorptive of light, or may scatter the light, not returning enough light to the optical light sensor 700 to generate a signal indicative of air being in the fluid path. If the valve diaphragm 170 is clear, then the retainer cap 190 may be clear, absorptive of light or may scatter the light, again not returning enough light to the optical light sensor 700 to generate a signal indicative of air being in the fluid path. If the liquid is opaque, it will absorb light. In any event, the light does not return to the photodetector. What little reflection of light may occur will be small compared to the case when air is present.

The material requirements of the preferred embodiment shown in Figure 108 are that the cassette body 100 be made of clear material, and that the valve diaphragm 170 be made of material which is clear, absorptive to light, or effectively scatters light. If the valve diaphragm 170 is clear, the retainer cap 190 must then be made of material which is clear, absorptive to light or effectively scatters light. In summary, the fluid channel in the assembled cassette 302 is designated so that with the presence of air in the fluid channel, light sent by the optical light source 698 will be detected by the optical light sensor 700. With liquid contained in the fluid channel, little or no light will be detected, irrespective of the clarity or opaqueness of the liquid. It will therefore be appreciated by those skilled in the art that air bubbles in the line may be easily detected with the apparatus discussed above.

There are three alternative embodiments to the arrangement illustrated in Figure 108. Firstly, in Figure 109, a reflective surface 702 is installed on the wall of the notch 680 opposite to the optical light source 698 and the optical light sensor 700. The recessed lens portion 138 in this embodiment is V-shaped, with light being directed from the bottom of the V. The materials of the cassette body 100, the valve diaphragm 170, and the retainer cap 190 are all clear. When a clear liquid is contained in the fluid pathway, light from the optical light source 698 will refract through to the reflective surface 702, and return to the optical light sensor 700, giving a high signal. When air is present in the fluid pathway, the light from the optical light source 698 will reflect off of the recessed lens portion 138 without passing though it, thereby not reaching the optical light sensor 700. However, when lipids are contained in the fluid pathway, the light will refract through the recessed lens portion 138 and be absorbed by the lipids, giving a signal indicative of air in the fluid pathway. It will therefore be appreciated that the arrangement shown in Figure 109 is suitable for use with clear liquids only.

Figure 110 shows a further variation which uses a V-shaped channel, with the bottom of the V being flat. Light is directed from the optical light source 698 which in this case is mounted in the top wall of the notch 680 directly opposite the optical light sensor 700 on the bottom wall of the notch 680. The materials of the cassette body 100, the valve diaphragm 170, and the retainer cap 190 are again clear. It will at once be appreciated that the signal received by the optical light sensor 700 will be low for lipids in the fluid pathway, and high for clear liquids in the fluid pathway. When air is present in the fluid pathway, some of the light will reflect off of the sides of the V, not reaching the optical light sensor 700, while some of the light will pass through the flat bottom of the V, reaching the optical light sensor 700. Therefore, for air a medium level signal will be received. The system of Figure 110 is accordingly a three level system and not digital.

Figure 111 shows a third variation which uses a V-shaped recessed lens portion 138, with light being directed from the top of the V. In this embodiment, the optical light source 698 and the optical light sensor 700 are mounted on the top wall of the notch 680, rather than in the bottom wall. The materials of the cassette body 100, the valve diaphragm 170, and the retainer cap 190 are again all clear. The signal received by the optical light sensor 700 will be high with air in the fluid pathway since light will be reflected by the surfaces of the V, low with clear liquids in the fluid pathway since light will be transmitted via the V, and generally medium with lipids contained in the fluid pathway. The system of Figure 111 is a three level system like the system of Figure 110, but the optics of the system of Figure 110 are superior to the optics of the system of Figure 111.

Figures 115 and 116 illustrate the operation of the pressure transducer system. The pressure diaphragm 182 contacts the bottom of the pressure transducer 660, which is flat. Additionally, the pressure diaphragm 182 does not contact the pressure plateau 130 either on it stop or sides, making movements of the pressure diaphragm 182 highly accurate and sensitive.

The pressure transducer 660 has a thin stainless steel diaphragm 710 at the bottom. The diaphragm 710 is supported at its edges by a stainless steel housing 712, which contains a passageway 714 leading to the square segment 664. The square segment 664 contains a sensor element (not shown in detail) communicating with the passageway 714, the sensor element being a standard silicon piezoresistive wheatstone bridge type device 716. The passageway 714 is filled with silicon oil to communicate pressure on the diaphragm 710 to the piezoresistive device 716.

It will be appreciated by those skilled in the art that the fluid pressure within the assembled cassette 302 will be communicated through the pressure diaphragm 182 and the diaphragm 710 to the silicone oil in the passageway 714, and thereby to the piezoresistive device 716, which provides an electrical indication of the pressure via the leads 666. Accordingly, pressure may be measured to provide an indication of downstream occulsion, pumping, fluid pressure etc.

Through the above discussion of the entire system, it will be appreciated that the disposable cassette 302 of the present invention retains all of the advantages of such devices known in the past, and in addition provides a number of additional advantages and improvements. The cassette 302 includes a pump affording a high degree of accuracy over a broad range of fluid flow rates, which may be conveniently and easily primed since it allows gravity flow of fluids through the cassette 302, and incudes a bubble trap to prevent the frequent shutdowns and alarms exhibited by presently known pumps. Accidental flow through the cassette when the cassette 302 is removed from the main pump unit is effectively prevented. The cassette 302 also includes both pressure sensing means and bubble detecting means in the cassette 302 itself rather than in peripheral devices.

Despite the inclusion of more features than contained in any known system, the cassette 302 of the present invention uses only seven parts, all of which are inexpensive yet highly precise in construction. The cassette 302 of the present invention is of a design and construction which enables it to compete economically with known systems, and yet provide an ease of installation and use rivaling the best of such known systems. It provides all of the advantages and features mentioned above, which are not all shown in any known cassette. The cassette 302 accomplishes all of these objectives and advantages in a manner which will retain all of the advantages of reliability, durability, and safety of operation, without incurring any relative disadvantage whatsoever.

**Claims**

1. A disposable cassette for use with a medication infusion system which comprises a cassette body (100) having an upper surface (102) with first and second apertures (110,118) extending therethrough, the upper surface (102) having an inlet passageway (128) and an outlet passageway (134); a cylinder (112) below the upper surface of the cassette, one end of the cylinder (112) being sealingly attached to the bottom of the upper surface (102), the other end of the cylinder (112) being open, the second aperture (118) being in communication with the interior of the cylinder (112); and diaphragm means (170) for sealing the open top sides of the inlet and outlet passageways (128,134), the diaphragm means (170) having a valve portion (178) which forms a cavity (180) over a portion of the upper surface of the cassette body, whereby the cavity (180) is in fluid communication with the inlet passageway (128), the valve portion (178) being selectively deformable to prevent the flow of fluids between the inlet passageway (128) and the second aperture (118); characterised in that: the inlet and outlet passageways (128,134) are recessed in the upper surface (102); the inlet passageway (128) extends between the first aperture (110) and a location near to the second aperture (118); the outlet passageway (134) extends between the location near to the second aperture (118) and a third aperture (142) in the upper surface (102); the inlet and outlet passageways (128,134) are open on the top side of the upper surface (102); a piston (260) is located in the cylinder (112) and is operable for varying the fluid volume contained in the cylinder (112) by alternatively drawing fluid into the cylinder (112) and forcing fluid out of the cylinder (112) through the second aperture (110); and cavity (180) extends over the said other end of the inlet passageway (128), the second aperture (118) and the said one end of the outlet passageway (134), whereby the cavity (180) is in the fluid communication with the second aperture (118) leading to the cylinder (112), and the outlet passageway (134); and the valve portion (178) is also selectively deformable to prevent the flow of fluids between the second aperture (128) and outlet passageway (134) during normal working of the system.

2. A cassette as claimed in Claim 1 characterised in that the valve portion (178) of the diaphragm means (170) comprises a flexible, resilient dome which forms the cavity (180).

3. A cassette as claimed in Claim 2, characterised in that the dome (178) is integral with the diaphragm means (170) the diaphragm means (170) being located around the dome (178).

4. A cassette as claimed in any preceding claim, characterised in that the cassette body (100) is of a one-piece moulded construction.

28

5. A cassette as claimed in any preceding claim, characterised by a bubble trap (104) disposed below the upper surface (102) of the cassette body (100), the bubble trap (104) preferably comprising a bubble chamber (106) located below the upper surface (102) of the cassette body (100) the bubble chamber (106) being open at the bottom; a siphon tube (108) located in the bubble chamber (106) extending from the bottom of the upper surface (102) to a position somewhat above the bottom of the bubble chamber (104), the siphon tube (108) having a bore (110) which is open at the bottom of the siphon tube (108) and communicates at the top with the first aperture (118); and a bubble chamber cap (230) for sealing the open bottom of the bubble chamber (106), the bubble chamber cap (230) including an inlet tube (304) extending up into the bubble chamber (106) from the bottom to a level above the bottom of the siphon tube (108).

6. A cassette as claimed in any preceding claim, characterised by a slightly raised border (146) which surrounds the inlet passageway (128), the second aperture (118) and the outlet passageway (134), and includes a rectangular portion spaced from and surrounding the said other end of the inlet passageway (128), the second aperture (118) and the said one end of the outlet passageway (134), the rectangular portion being broken only at the locations where the inlet passageway (128) and the outlet passageway (134) extend out of the rectangular portion, the border (146) co-operating with the diaphragm means (170) for sealing purposes.

7. A cassette as claimed in any preceding claim, characterised by a first raised segment (147) located between the said other end of the inlet passageway (128) and the second aperture (118) and a second raised segment (149) located between the said one end of the outlet passageway (134) and the second aperture (118); the two raised segments (147,149) being located below the valve portion (178) and being wider than the cavity (180); the first raised segment (147) cooperating with the valve portion (178) to prevent selectively the flow of fluids between the inlet passageway (128) and the second aperture (118), and the second raised segment (149) cooperating with the valve portion (178) to prevent selectively the flow of fluids between the second aperture (118) and the outlet passageway (134).

8. A cassette as claimed in any preceding claim, characterised by means for selectively preventing the flow of fluid through the cassette prior to its installation, for example to avoid inadvertent flow, the preventing means being operable to allow the flow of fluid through the cassette prior to installation in order to prime the cassette, the preventing means preferably comprising an outlet tube (306) for receiving fluid pumped form the cassette body; the outlet tube (306) being fixedly attached to the cassette body by outlet tube mounting means (144) located below the upper surface (102) of the cassette body, the outlet tube (306) having one end communicating with the third aperture (148) in order to receive the fluid; and means for selectively clamping the outlet tube to prevent fluid from flowing.

9. A cassette as claimed in Claim 8 characterised in that the clamping means comprises: a slide latch (240) having a tear-shaped aperture (258) the tear-shaped aperture (258) having a wider end and a narrower end; and slide latch support means located below the upper surface of the cassette body, supporting the slide latch (240) for slideable movement with the tear-shaped aperture (258) located under the outlet tube mounting means (144), the outlet tube extending (306) through the tear-shaped aperture (258), the slide latch (240) having a first position in which the outlet tube (306) extends through the wider end of the tear-shaped aperture (258) allowing fluid to flow through the outlet tube (306), the slide latch (240) also having a second position in which the outlet tube (306) extends through the narrower end of the tear-shaped aperture (258) thereby constricting the outlet tube (306) to prevent fluid from flowing though the outlet tube (306).

10. A cassette as claimed in any preceding claim, characterised by a retainer cap (190) for installation over the upper surface of the diaphragm means (170) and cassette body (100), the retainer cap (190) retaining the diaphragm means (170) in a sealing relationship over the upper surface of said cassette body (100), the retainer cap (190) having an aperture (212) allowing the valve portion (178) of the diaphragm means (170) to project therethrough, permitting access to the valve portion (178) so that it may be operated.

11. A cassette as claimed in any preceding claim, characterised in that the inlet and outlet passageways (128,134) are essentially U-shaped in section.

## Patentansprüche

1. Einmal-Kassette zum Gebrauch bei einem Medikamenten-Infusionssystem, mit einem Kassettenkörper (100), der eine obere Fläche (102) mit ersten und zweiten, durch sie hindurchlaufenden Öffnungen (110, 118) sowie mit einem Einlaßkanal (128) und einem Auslaßkanal (134) aufweist, mit einem Zylinder (112) unter der oberen Fläche der Kassette, wobei ein Ende des Zylinders (112) dicht an der Unterseite der oberen Fläche (102) befestigt und das andere Ende des Zylinders (112) offen ist und die zweite Öffnung (118) in Verbindung mit dem Inneren des Zylinders (112) steht, und mit Membraneinrichtungen (170) zum Abdichten der offenen Oberseiten des Einlaß- und des Auslaßkanals (128, 134), wobei die Membraneinrichtungen (170) einen Ventilabschnitt (178) aufweisen, der einen Hohlraum (180) über einem Bereich der oberen Fläche des Kassettenkörpers ausbildet, wodurch der Hohlraum (180) in Strömungsverbindung mit dem Einlaßkanal (128) steht, und wobei der Ventilabschnitt (178) wahlweise verformbar ist, um das Fließen von Fluid zwischen dem Einlaßkanal (128) und der zweiten Öffnung (118) zu verhindern, **dadurch gekennzeichnet,** daß der Einlaß- und der Auslaßkanal (128, 134) in die obere Fläche (102) eingelassen sind, der Einlaßkanal (128) zwischen der ersten Öffnung (110) und einer Stelle nahe der zweiten Öffnung (118) verläuft, der Auslaßkanal (134) zwischen der Stelle nahe der zweiten Öffnung (118) und einer dritten Öffnung (142) in der oberen Fläche (102) verläuft, der Einlaß- und der Auslaßkanal (128, 134) an der Oberseite der oberen Fläche (102) offen sind, ein Kolben (260) im Zylinder (112) angeordnet und zur Veränderung des im Zylinder (112) enthaltenen Fluidvolumens betätigbar ist, indem der Kolben abwechselnd Fluid in den Zylinder (112) zieht und aus dem Zylinder (112) durch die zweite Öffnung (118) herausdrückt, daß sich ferner der Hohlraum (180) über das andere Ende des Einlaßkanals (128), die zweite Öffnung (118) und das eine Ende des Auslaßkanals (134) erstreckt, wodurch der Hohlraum (180) in Strömungsverbindung mit der zweiten, zum Zylinder (112) führenden Öffnung (118) und dem Auslaßkanal (134) steht, und daß der Ventilabschnitt (178) ebenfalls wahlweise verformbar ist, um das Fließen von Fluid zwischen der zweiten Öffnung (118) und dem Auslaßkanal (134) während des Normalbetriebs des Systems zu verhindern.

2. Kassette nach Anspruch 1, dadurch gekennzeichnet, daß der Ventilabschnitt (178) der Membraneinrichtungen (170) eine flexible, elastische Kuppel aufweist, die den Hohlraum (180) ausbildet.

3. Kassette nach Anspruch 2, dadurch gekennzeichnet, daß die Kuppel (178) einstückig mit den Membraneinrichtungen (170), die um die Kuppel (178) herum angeordnet sind, ausgebildet ist.

4. Kassette nach einem der vorhergehenden Ansprüche, dadurch gekennzeichnet, daß der Kassettenkörper (100) als eine einteilig geformte Konstruktion ausgebildet ist.

5. Kassette nach einem der vorhergehenden Ansprüche, gekennzeichnet durch einen Blasenabscheider (104), der unter der oberen Fläche (102) des Kassettenkörpers (100) angeordnet ist und vorzugsweise eine unter der oberen Fläche (102) des Kassettenkörpers (100) angeordnete Blasenkammer (106) aufweist, die an ihrem unteren Ende offen ist, ferner durch ein in der Blasenkammer (106) angeordnetes Siphonrohr (108), das vom Boden der oberen Fläche (102) zu einer Stelle etwas oberhalb des unteren Endes der Blasenkammer (106) verläuft und eine Bohrung (110) aufweist, die am unteren Ende des Siphonrohrs (108) offen ist und am oberen Ende mit der ersten Öffnung (110) in Verbindung steht, und durch eine Blasenkammer-Kappe (230) zum Abdichten des offenen unteren Endes der Blasenkammer (106), wobei die Blasenkammer-Kappe (230) einen Einlaßschlauch (304) aufweist, der sich in die Blasenkammer (106) von deren unterem Ende aus bis zu einer Höhe oberhalb des unteren Endes des Siphonrohrs (108) erstreckt.

6. Kassette nach einem der vorhergehenden Ansprüche, gekennzeichnet durch einen leicht erhöhten Rand (146), der den Einlaßkanal (128), die zweite Öffnung (118) sowie den Auslaßkanal (134) umgibt und einen rechteckigen Bereich umfaßt, der in einem Abstand vom anderen Ende des Einlaßkanals (128), der zweiten Öffnung (118) und dem einen Ende des Auslaßkanals (134) angeordnet ist sowie diese umgibt und lediglich an den Stellen unterbrochen ist, an denen der Einlaßkanal (128) und der Auslaßkanal (134) aus dem rechteckigen Bereich hinauslaufen, wobei der Rand (146) mit den Membraneinrichtungen (170) zum Zweck einer Abdichtung zusammenwirkt.

7. Kassette nach einem der vorhergehenden Ansprüche, gekennzeichnet durch einen ersten erhöhten Abschnitt (147), der zwischen dem anderen Ende des Einlaßkanals (128) und der zweiten Öffnung (118) angeordnet ist, und durch einen zweiten erhöhten Abschnitt (149), der zwischen dem einen Ende des Auslaßkanals (134) und der zweiten Öffnung (118) angeordnet ist, wobei die beiden erhöhten Abschnitte (147, 149) unter dem Ventilabschnitt (178) angeordnet sind und weiter als der Hohlraum (180) auseinanderliegen, und wobei der erste erhöhte Abschnitt (147) mit dem Ventilabschnitt (178) zusammenwirkt, um wahlweise das Fließen von Fluid zwischen dem Einlaßkanal (128) und der zweiten Öffnung (118) zu verhindern, während der zweite erhöhte Abschnitt (149) mit dem Ventilabschnitt (178) zusammenwirkt, um wahlweise das Fließen von Fluid zwischen der zweiten Öffnung (118) und dem Auslaßkanal (134) zu verhindern.

8. Kassette nach einem der vorhergehenden Ansprüche, gekennzeichnet durch Mittel zum wahlweisen Verhindern des Fließens von Fluid durch die Kassette vor deren Einsetzen, z.B. zur Vermeidung eines versehentlichen Fließen, wobei diese Verhinderungsmittel betätigt werden können, um zur Inbetriebnahme der Kassette vor deren Einsetzen das Fließen von Fluid durch die Kassette zu ermöglichen, und vorzugsweise einen Auslaßschlauch (306) zur Aufnahme von Fluid aufweisen, das vom Kassettenkörper abgepumpt wird, wobei der Auslaßschlauch (306) durch Befestigungsmittel (144), die unter der oberen Fläche (102) des Kassettenkörpers angeordnet sind, fest am Kassettenkörper befestigt ist und mit einem Ende mit der dritten Öffnung (142) zur Aufnahme des Fluids in Verbindung steht, und durch Mittel zum wahlweisen Abklemmen des Auslaßschlauchs, um ein Fließen von Fluid zu verhindern.

9. Kassette nach Anspruch 8, dadurch gekennzeichnet, daß die Klemmeinrichtung folgende Merkmale umfaßt: eine Gleitraste (240) mit einer tropfenförmigen Öffnung (258), die ein breiteres und ein schmaleres Ende aufweist, Stützmittel für die Gleitraste, die unter der oberen Fläche des Kassettenkörpers angeordnet sind und die Gleitraste (240) mit der tropfenförmigen Öffnung (258), die unter den Befestigungsmitteln (144) für den Auslaßschlauch angeordnet ist, gleitbeweglich abstützen, wobei der Auslaßschlauch (306) durch die tropfenförmige Öffnung (258) verläuft und die Gleitraste (240) eine erste Stellung, in welcher der Auslaßschlauch (306) durch das breitere Ende der tropfenförmigen Öffnung (258) verläuft, um ein Fließen von Fluid durch ihn hindurch zuzulassen, sowie eine zweite Stellung aufweist, bei welcher der Auslaßschlauch (306) durch das schmalere Ende der tropfenförmigen Öffnung (258) verläuft, wodurch er eingeschnürt wird, um ein Fließen von Fluid durch ihn hindurch zu verhindern.

10. Kassette nach einem der vorhergehenden Ansprüche, gekennzeichnet durch eine Haltekappe (190) zum Einbau über der oberen Fläche der Membraneinrichtungen (170) und des Kassettenkörpers (100), wobei die Haltekappe (190) die Membraneinrichtungen (170) in dichtender Weise über der oberen Fläche des Kassettenkörpers (100) hält und eine Öffnung (212) zum Durchragen des Ventilabschnitts (178) der Membraneinrichtung (170) aufweist, wodurch der Ventilabschnitt (178) zugänglich wird, so daß er betätigt werden kann.

11. Kassette nach einem der vorhergehenden Ansprüche, dadurch gekennzeichnet, daß der Einlaß- und der Auslaßkanal (128, 134) einen im wesentlichen U-förmig ausgebildeten Querschnitt aufweisen.

## Revendications

1. Cassette jetable destinée à être utilisée avec un système de perfusion d'un médicament, comprenant un corps de cassette (100) comportant une surface supérieure (102) et des première et deuxième lumières (110, 118) qui le traverse, la surface supérieure (102) comportant un passage d'entrée (128) et un passage de sortie (134) ; un cylindre (112) sous la surface supérieure de la cassette, une extrémité du cylindre (112) étant assujettie de manière étanche au fond de la surface supérieure (102), l'autre extrémité du cylindre (112) étant ouverte, la deuxième lumière (118) étant en communication avec l'intérieur du cylindre (112) ; et un moyen à diaphragme (170) pour fermer de manière étanche les côtés supérieurs ouverts des passages d'entrée et de sortie (128, 134), le moyen à diaphragme (170) comprenant une partie (178) constituant un clapet et formant une cavité (180) sur une partie de la surface supérieure du corps de cassette, la cavité (180) étant en communication fluidique avec le passage d'entrée (128), la partie (178) constituant un clapet étant sélectivement déformable pour empêcher l'écoulement de fluide entre le passage d'entrée (128) et la deuxième lumière (118) ; caractérisée en ce que les passages d'entrée et de sortie (128, 134) sont formés d'évidements dans la surface supérieure (102) ; le passage d'entrée (128) est disposé entre la première lumière (110) et un emplacement proche de la deuxième lumière (118) ; le passage de sortie (134) est disposé entre l'emplacement proche de la deuxième lumière (118) et une troisième lumière (142) réalisée dans la surface supérieure (102) ; les passages d'entrée et de sortie (128, 134) sont ouverts sur le côté supérieur de la surface supérieure (102) ; un piston (260) est placé dans le cylindre (112) et capable de faire varier le volume de fluide que contient le cylindre (112) en provoquant en alternance l'aspiration de fluide dans le cylindre (112) et le refoulement de fluide hors du cylindre (112) par la deuxième lumière (118) ; et une cavité (180) est située sur ladite autre extrémité du passage d'entrée (128), la deuxième lumière (118) et ladite une extrémité du passage de sortie (134), de manière que la cavité (180) soit en communication fluidique avec la deuxième lumière (118) qui débouche dans le cylindre (112) et avec le passage de sortie (134) ; et la partie (178) constituant un clapet étant aussi déformable sélectivement pour empêcher l'écoulement de fluide entre la deuxième lumière (128) et le passage de sortie (134) pendant le fonctionnement normal du système.

2. Cassette selon la revendication 1, caractérisée en ce que la partie (178) du moyen à diaphragme (170) qui constitue un clapet comporte un dôme souple et élastique qui forme la cavité (180).

3. Cassette selon la revendication 2, caractérisée en ce que le dôme (178) est monobloc avec le moyen à diaphragme (170), le moyen à diaphragme (170) étant placé autour du dôme (178).

4. Cassette selon l'une quelconque des revendications précédentes, caractérisée en ce que le corps de cassette (100) est d'un mode d'exécution moulé en une pièce.

5. Cassette selon l'une quelconque des revendications précédentes, caractérisée par un piège à bulles (104) disposé sous la surface supérieure (102) du corps (100) de la cassette, le piège à bulles (104) comprenant de préférence une chambre à bulles (106) située sous la surface supérieure (102) du corps (100) de la cassette, la chambre à bulles (106) étant ouverte au fond ; un tube de siphon (108) placé dans la chambre à bulles (106) allant du fond de la surface supérieure (102) à un emplacement se trouvant légèrement au-dessus du fond de la chambre à bulles (104), le tube de siphon (108) ayant un trou (110) qui est ouvert au fond du tube de siphon (108) et communique au sommet avec la première lumière (118) ; et un capuchon (230) de chambre à bulles pour fermer de manière étanche le fond ouvert de la chambre à bulles (106), le capuchon (230) de la chambre à bulles comprenant un tube d'entrée (304) allant de bas en haut dans la chambre à bulles (106) et atteignant un niveau situé au-dessus du bas du tube de siphon (108).

6. Cassette selon l'une quelconque des revendications précédentes, caractérisée par un rebord (146) légèrement surélevé qui entoure le passage d'entrée (128), la deuxième lumière (118) et le passage de sortie (134) et qui comprend une partie rectangulaire placée à distance de et entourant ladite autre extrémité du passage d'entrée (128), la deuxième lumière (118) et ladite une extrémité du passage de sortie (134), ladite partie rectangulaire n'étant interrompue qu'aux emplacements auxquels le passage d'entrée (128) et le passage de sortie (134) sortent de la partie rectangulaire, le rebord (146) coopérant avec le moyen à diaphragme (170) à des fins d'étanchéïté.

7. Cassette selon l'une quelconque des revendications précédentes, caractérisée par un premier segment surélevé (147) placé entre ladite autre extrémité du passage d'entrée (128) et la deuxième lumière (118) et un second segment surélevé (149) situé entre ladite une extrémité du passage de sortie (134) et la deuxième lumière (118) ; les deux segments surélevés (147, 149) étant placés sous la partie (178) constituant un clapet et étant plus larges que la cavité (180) ; le premier segment surélevé (147) coopérant avec la partie (178) constituant un clapet pour empêcher sélectivement l'écoulement de fluide entre le passage d'entrée (128) et la deuxième lumière (118) et le second segment surélevé (149) coopérant avec la partie (178) constituant un clapet pour empêcher sélectivement l'écoulement de fluide entre la deuxième lumière (118) et le passage de sortie (134).

8. Cassette selon l'une quelconque des revendications précédentes, caractérisée par des moyens d'inhibition sélective de l'écoulement de fluide par la cassette avant sa mise en place, par exemple pour éviter un écoulement intempestif, les moyens d'inhibition étant capables de permettre l'écoulement de fluide par la cassette avant la mise en place afin d'amorcer la cassette, les moyens d'inhibition comprenant de préférence un tube de sortie (306) destiné à réceptionner le fluide pompé sur le corps de la cassette ; le tube de sortie (306) étant fixé de manière inamovible au corps de la cassette par des moyens (144) de montage du tube de sortie situés sous la surface supérieure (102) du corps de la cassette, le tube de sortie (306) ayant une extrémité qui communique avec la troisième lumière (148) afin de recevoir le fluide ; et des moyens pour pincer sélectivement le tube de sortie afin d'empêcher l'écoulement de fluide.

9. Cassette selon la revendication 8, caracérisée en ce que les moyens de pinçage comprennent : un loquet coulissant (240) comportant un trou en forme de larme (258), le trou en forme de larme (258) comprenant une extrémité large et une extrémité étroite ; des moyens de support du loquet coulissant situés sous la surface supérieure de corps de cassette, supportant le loquet coulissant (240) de manière à lui permettre de coulisser, le trou en forme de larme (258) étant placé sous les moyens (144) de montage du tube de sortie, le tube de sortie passant (306) par le trou (258) en forme de larme, le loquet coulissant (240) pouvant occuper une première position dans laquelle le tube de sortie (306) passe par l'extrémité large du trou en forme de larme (258) de manière à permettre au fluide de s'écouler par le tube de sortie (306), le loquet coulissant (240) pouvant aussi occuper une deuxième position dans laquelle le tube de sortie (306) passe par l'extrémité étroite du trou en forme de larme (258) de manière à étrangler le tube de sortie (306) pour empêcher le fluide de s'écouler par le tube de sortie (306).

10. Cassette selon l'une quelconque des revendications précédentes, caractérisée par une coiffe de retenue (190) destinée à être mise en place sur la surface supérieure du moyen à diaphragme (170) et sur le corps (100) de la cassette, la coiffe de retenue (190) retenant le moyen à diaphragme (170) de manière étanche sur la surface supérieure du corps (100) de la cassette, la coiffe de retenue (190) comportant une ouverture (212) permettant à la partie (178) du moyen à diaphragme (170) qui constitue un clapet de passer par elle afin de permettre l'accès à la partie (178) constituant un clapet de façon qu'elle puisse être actionnée.

11. Cassette selon l'une quelconque des revendications précédentes, caractérisée en ce que les passages d'entrée et de sortie (128, 134) ont sensiblement une forme U en coupe transversale.

*FIG.1*

*FIG.2*

*FIG.3*

FIG. 6

FIG. 7

FIG. 4

FIG. 8

FIG. 5

FIG.9

FIG.11

FIG.22

FIG.24

FIG.12

FIG.25

FIG.23

FIG.10

FIG.26

36

FIG. 19

FIG. 21

FIG. 14

FIG. 20

FIG. 15

FIG. 18

FIG. 13

FIG. 17

FIG. 16

Fig.27

Fig.28

Fig.31

Fig.29

Fig.30

Fig.32

Fig.34

Fig.35

272

270

276

260  266

FIG.33

272

270

262

268

276

274

264

260  266

FIG.36

300

296

298

284

290

280  282

FIG.39

296

294

292

298

280  282

290

286

FIG.40

282

300

292

294

296

284

286

290

280

FIG.42

290  286  282

298

300

296  294  292  288

280

FIG.37

284

290

282  280

FIG.41

286

298

300  292

284  288

282  290  296

280

FIG.38

39

FIG. 46

FIG. 45

FIG. 47

FIG. 43

FIG. 48

FIG. 44

FIG. 49

FIG. 51

FIG. 50

FIG. 52

FIG. 53

FIG. 54

FIG. 55

FIG. 56

FIG. 57

FIG. 58

FIG. 59

FIG_60

FIG_61

FIG_66

FIG_69

FIG_67

FIG_68

FIG_70

FIG.64

FIG.63

FIG.62

FIG.65

FIG.71

FIG.72

FIG.73

FIG.74

FIG.75

FIG.76

FIG.77

FIG.78

FIG.79

FIG.80

FIG-81

FIG-82

FIG-85

FIG-86

FIG-87

FIG-83

FIG-84

FIG-88

FIG-89

FIG-90

FIG.91

FIG.92

FIG.99

FIG.93

FIG.94

FIG.100

686 688
690
672
670
FIG.95

700 692
698 694 696
670
FIG.96

650
652 654
FIG.98

652
654
98 98
650
FIG.97

FIG.101

FIG.102

670 680
106
522 520
684 682
510 562
560 570 566 530 532 510
568
106
FIG.105

674
688
680 190
208 100
150 694
670
FIG.103

674
690 190
680 152
210 100
696
670
FIG.104

670
522 682
570
510 568
FIG.106

48

*Fig. 107*

*Fig. 108*

*Fig. 110*

*Fig. 109*

*Fig. 111*

FIG. 115

FIG. 116

FIG. 112

50

Fig. 113

Fig. 114

EP 0 319 278 B1